# EUROPEAN PATENT APPLICATION

(11) **EP 4 245 319 A1**
(43) Date of publication of application: **20.09.2023**
(21) Application number: 21892337.3
(22) Date of filing: 11.11.2021
(51) Int. Cl.: A61K 47/59, A61P 35/00, C07K 16/28, A61K 39/00

(54) **ANTIBODY-BASED CONJUGATE FOR ENHANCING THERAPEUTIC EFFECT OF TARGETED THERAPEUTIC AGENT**

(30) Priority: 11.11.2020 KR 20200150142; 14.09.2021 KR 20210122669
(71) Applicant: The Catholic University of Korea Industry-Academic Cooperation Foundation, Seoul (KR)
(72) Inventor: NA, Kun, Bucheon-si, Gyeonggi-do 14574 (KR); KIM, Da Hye, Seoul 07372 (KR); AHN, Min Ji, Seoul 08012 (KR); PARK, Na Eun, Incheon 21977 (KR)
(74) Representative: Plasseraud IP
(86) International application number: PCT/KR2021/016438
(87) International publication number: WO 2022/103170

(57) **Abstract**

The present invention relates to a conjugate including an antibody for treating cancer, a linker, and a block copolymer including PEO and PPO, and a conjugate further including a low molecular weight compound. The conjugate has excellent cancer cell targeting ability and can efficiently increase cancer cells by increasing the half-life of the antibody, and thus can be effectively used for treating cancer.

## Description

### [Technical Field]

The present invention relates to a conjugate capable of enhancing the therapeutic effect of a targeted therapeutic agent, including an antibody, a linker, and a block copolymer including poly(ethylene oxide) (PEO) and poly(propylene oxide) (PPO), and a conjugate in which a low molecular compound further binds to the above conjugate.

### [Background Art]

Since traditional chemotherapy is one of the essential methods for treating cancer and most chemotherapy anticancer agents used in anticancer targets the cell cycle, the toxicity depends on the degree of proliferation of cancer cells. Further, chemotherapy anticancer agents are generally used near the maximum tolerated dose to obtain the clinical therapeutic effect, and treatment using various drugs has become the standard treatment therapy for cancer treatment. However, anticancer agents only kill rapidly proliferating cells and cannot differentiate between normal cells and tumor cells or tumor tissue. Due to these disadvantages, systemic toxicity and cytotoxicity occur, and long-term treatment may cause resistance to anticancer agents, and therefore there is a desperate need for improved therapies that target and kill only cancer cells with cytotoxic drugs.

Unlike cytotoxic drugs, monoclonal antibodies that bind to specific antigens on the surfaces of tumor cells are an alternative treatment therapy that reduces systemic toxicity because the monoclonal antibodies bind specifically to tumors. In fact, antigens preferentially or exclusively expressed on the surfaces of cancer cells have been identified through expression profiling studies, and monoclonal antibodies specifically binding to tumor-associated antigens may be engineered and produced. A certain form of targeted treatment in the form of a drug at the molecular level prevents cancer cells from proliferating by blocking signals involved in carcinogenesis and tumor growth. Cancer cell-targeted treatment methods are expected to be more effective methods than existing methods while not harming normal cells. Such monoclonal antibodies are under continuous development, and there are already therapeutic agents approved by the US Food and Drug Administration (FDA). Examples of approved monoclonal antibodies include rituximab, trastuzumab, alemtuzumab, cetuximab, bevacizumab, ipilimumab, and the like.

However, only a few antibodies are useful for cancer treatment because most antibodies are not very efficient at killing cancer cells. In order for antibody-based targeted anticancer agents to be efficiently used, there is a need to induce effective cancer cell killing by a single injection and maintain antigen-antibody binding with a strong binding force for a long period of time.

### [Disclosure]

### [Technical Problem]

Under these circumstances, the present inventors have conducted research on a method which can be efficiently used by inducing antibody-based targeted anticancer drugs to effectively kill cancer cells with only a single injection and maintaining antigen-antibody binding with a strong binding force for a long period of time.

As a result, the present inventors prepared a conjugate in which a linker and a block copolymer including PEO and PPO are linked to a monoclonal antibody selectively binding to a targeted factor overexpressed in cancer cells, and confirmed that such conjugate can remain in the body longer and induce better anti-cancer effects than existing therapeutic agents through interactions with cell membranes.

In addition, the present inventors additionally prepared a conjugate in which a low molecular weight compound was conjugated to one end of the above conjugate, and confirmed that the additionally prepared conjugate overcomes both the limitations of monoclonal antibodies and the limitations of low molecular weight compounds, and can induce a better anticancer effect due to a synergistic effect of a block copolymer including PEO and PPO, thereby completing the present invention.

Therefore, an object of the present invention is to provide an anticancer-based conjugate for treating cancer with enhanced *in vivo* half-life and therapeutic effect, and a pharmaceutical composition for treating cancer, including the same.

### [Technical Solution]

To achieve the object, an aspect of the present invention provides a conjugate including: (a) an antibody for treating cancer; (b) a linker linked to the antibody via a covalent bond; and (c) a block copolymer including PEO and PPO, which are linked to the linker via a covalent bond.

As used herein, the term "antibody for cancer therapy" refers to an antibody used for treating cancer, and may be distinguished according to the origin of the antibody as an animal-derived antibody, a chimeric antibody, a humanized antibody, or a human antibody.

An animal-derived antibody is an antibody produced by injecting an antigen into a non-human animal, and a chimeric antibody is an antibody in which the constant region of an animal-derived antibody which induces the most immunogenicity is replaced with that of a human antibody.

A humanized antibody is an antibody in which the sequence of the rest of the animal-derived antibody, excluding the complementarity determining region (CDR) sequence, which is an antigen-binding site, is substituted with a human antibody sequence in order to eliminate the immunogenicity of the animal-derived antibody.

Furthermore, a human antibody is an antibody produced by selecting an antibody against a specific antigen by a phage display technique of a human antibody library and then introducing the corresponding antibody gene into mice.

Further, antibodies for treating cancer may be classified according to the action mechanism thereof into receptor-targeting antibodies and immune checkpoint inhibitors or immune checkpoint blockades.

A receptor-targeting antibody is an antibody that specifically binds to a specific receptor on the surfaces of cancer cells. According to an exemplary embodiment of the present invention, the specific receptor may be selected from the group consisting of an epidermal growth factor receptor (EGFR), human epidermal growth factor receptor 2 (HER2) and vascular endothelial growth factor receptor 2 (VEGFR2).

Immune checkpoint inhibitors maintain the immune function of T cells by hindering programmed death-ligand 1 (PD-L1) of cancer cells from binding to PD-1 of T cells.

The epidermal growth factor receptor (EGFR) is a group of cell membrane receptors that regulate the growth, division and death of cells. The epidermal growth factor receptor (EGFR) is a type 1 membrane protein with 170 kDa, which is known to be overexpressed in various types of tumors (solid tumors such as lung cancer, head and neck tumors, colorectal cancer, pancreatic cancer, and breast cancer) due to the amplification and expression of the receptor. Tumor tissue in which the epidermal growth factor receptor is overexpressed tends to be more invasive, more metastatic, more resistant to anticancer therapies, and thus has a poorer prognosis Targeted therapies using antibodies that target the epidermal growth factor receptor have been conducted to overcome this. Antibodies bind to epidermal growth factor receptors to inhibit the epidermal growth factors from binding, thereby suppressing the signaling and growth of cancer cells to treat cancer.

Cetuximab, which is a type of chimeric antibody, binds to an epidermal growth factor receptor (EGFR) on the cell surface to interfere with the binding of ligands, thereby inhibiting receptor activation, increasing the internalization of the receptor into cells, and decreasing the expression of the receptor. As a result, cetuximab arrests the cell cycle at G0-G1, induces the dephosphorylation of the Rb gene, inhibits cell proliferation, induces cell apoptosis, and suppresses the production of angiogenic factors such as VEGF.

Human epidermal growth factor receptor 2 (HER2) is a tyrosine-phosphorylated growth factor receptor with a molecular weight of 185 kDa, which is present on the cell surface. Although there is no ligand-binding site in the HER2 molecule, HER2 is activated by easily dimerizing with other receptors such as EGFR, HER3 and HER4. Receptor-ligand conjugations exhibit effects such as cell proliferation, cell survival, metastasis, and angiogenesis through various cell signaling pathways. HER2 is overexpressed in various cancer types, such as 20 to 30% of breast cancer, gastric cancer, ovarian cancer, lung cancer, and prostate cancer. The overexpression thereof further enhances the function of promoting the survival, proliferation, angiogenesis and metastasis of cells.

Trastuzumab, which is a humanized antibody, is a recombinant human monoclonal antibody that targets the extracellular domain site of the HER2 protein, and was the first to be approved by the FDA. By suppressing signaling pathways in HER2-overexpressing tumor cells and suppressing the intracellular G1/S cell cycle, cell apoptosis is induced, and susceptibility to anticancer agents such as platinum agents, taxane, doxorubicin, and cyclophosphamide is increased. In addition, when an antibody binds to the extracellular region of HER2, HER2 receptors are reduced.

Programmed death ligand 1 (PD-L1) is a type 1 transmembrane protein with 40 kDa, and a type of protein abundantly expressed in cancer cells. PD-L1 serves to evade the attack of immune cells by interacting with PD-1 receptors present on the surface of T cells. Their interaction simultaneously reduces the cell apoptosis of regulatory cells while reducing the proliferation of antigen-specific T cells in lymph nodes, allowing cancer cells to evade anticancer immune responses.

Avelumab, which is a type of human antibody, is an immune checkpoint inhibitor, and is a human monoclonal antibody that targets PD-L1 overexpressed in cancer cells. This antibody activates the immunosuppressive environment formed in cancer cells by preventing the interaction of PD-1 of T cells and PD-L1 of cancer cells. Avelumab has an advantage in that the effect against cancer in which PD-L1 is expressed regardless of the type of cancer can be exhibited by blocking the activity of the PD-L1 protein, which acts as an immune checkpoint. Avelumab is known to be effective against non-small cell lung cancer, melanoma, colorectal cancer, kidney cancer, hepatocellular carcinoma, and the like.

Vascular endothelial growth factor receptor 2 (VEGFR2) is expressed in vascular and lymphatic endothelial cells and binds to VEGF-A, VEGF-E, and the like to promote vascular proliferation and vascular endothelial cell migration.

Ramucirumab, which is a type of human antibody, is a VEGFR2 antagonist, blocks ligand binding, and consequently suppresses receptor activation, and is used to treat colorectal cancer, non-small lung cancer and gastric cancer.

As used herein, the term "linker" refers to a material that links an antibody for targeting cancer to a poly(ethylene oxide)-poly(propylene oxide)-poly(ethylene oxide) (PEO-PPO-PEO) block copolymer. According to an exemplary embodiment of the present invention, the linker may be selected from the group consisting of maleimide, succinic anhydride and N-hydroxysuccinimide ester, and may be preferably maleimide or succinic anhydride.

As used herein, the term "block copolymer including PEO and PPO (hereinafter referred to as PEO-PPO block copolymer)" refers to a copolymer alternately including a polypropylene oxide block and a polyethylene oxide block.

According to an exemplary embodiment of the present invention, the PEO-PPO block copolymer may be a PEO-PPO-PEO block copolymer which is a triple copolymer alternately including a polypropylene oxide block and a polyethylene oxide block.

According to an exemplary embodiment of the present invention, the PEO-PPO block copolymer may be selected from the group consisting of poloxamer 68, poloxamer 124, poloxamer 127, poloxamer 184, poloxamer 185, poloxamer 188, poloxamer 237, poloxamer 338 and poloxamer 407. Preferably, the PEO-PPO-PEO block copolymer may be poloxamer 188 (Pluronic^{®} F-68) or poloxamer 407 (Pluronic^{®} F-127).

In the present invention, the antibody for treating cancer and the linker, and the linker and the PEO-PPO-PEO block copolymer may each be linked via a covalent bond. The covalent bond may be selected from the group consisting of an amide bond, a carbonyl bond, an ester bond, a thioester bond, a sulfonamide bond and a urethane bond.

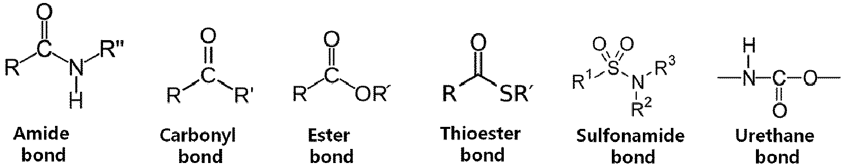

In an exemplary embodiment of the present invention, the conjugate may be prepared by a method of first combining a linker and a PEO-PPO block copolymer, and then further combining an antibody for targeting cancer, or first combining a cancer-targeting antibody and a linker and combining a PEO-PPO block copolymer.

Further, according to an exemplary embodiment of the present invention, the conjugate may further include a low molecular weight compound at one end thereof, and preferably, a low molecular weight compound may bind to the end of the PEO-PPO block copolymer.

In the present invention, the low molecular weight compound may be an anticancer agent or a photosensitizer, the anticancer agent may be a cytotoxic anticancer agent, and the photosensitizer may be selected from the group consisting of chlorins, bacteriochlorins, porphyrins, porphycenes and phthalocyanine. For example, meso tetra aminophenyl porphyrin, zinc protoporphyrin, protoporphyrin, and hemato porphyrin may be used as a porphyrin photosensitizer, and aluminum phthalocyanine may be used as a phthalocyanine photosensitizer.

According to an exemplary embodiment of the present invention, the chlorin photosensitizer may be chlorin e6. The chlorin e6 may bind to the end of the PEO-PPO block copolymer as already mentioned.

The present inventors confirmed that a conjugate in which an antibody for targeting cancer, a linker, and a PEO-PPO block copolymer are linked enhances the ability of the antibody to target cancer cells to induce effective cell apoptosis and enhance the *in vivo* half-life of the antibody per se. Furthermore, they confirmed that cancer cells can be efficiently killed because a conjugate to which a low molecular weight compound further binds exhibits the effect of killing cancer cells due to photosensitizers in addition to the above-mentioned effect.

Therefore, another aspect of the present invention provides a pharmaceutical composition for treating cancer, including the conjugate as an effective ingredient. Since the antibody for treating cancer-linker-PPO block copolymer conjugate or the antibody for treating cancer-linker-PEO-PPO block copolymer-low molecular weight compound conjugate is used as an effective ingredient in the pharmaceutical composition, the description of overlapping content between the two will be omitted to avoid the excessive complication of the specification.

The pharmaceutical composition of the present invention may include a pharmaceutically acceptable carrier in addition to the active ingredient. In this case, the pharmaceutically acceptable carrier is one typically used during formulation, and examples thereof include lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, mineral oil, and the like, but are not limited thereto. Furthermore, the pharmaceutically acceptable carrier may further include a lubricant, a wetting agent, a sweetening agent, a flavoring agent, an emulsifier, a suspending agent, a preservative, and the like in addition to the above components.

The pharmaceutical composition of the present invention may be administered orally or parenterally (for example, intravenously, subcutaneously, or intraperitoneally or applied topically) according to the desired method. When the active ingredient of the present invention is formulated into a preparation such as tablets, capsules, chewable tablets, a powder, a liquid, and a suspending agent for the purpose of oral administration, it is possible to include a binder such as arabic rubber, corn starch, microcrystalline cellulose or gelatin, an excipient such as calcium diphosphate or lactose, a disintegrant such as alginic acid, corn starch, or potato starch, a lubricant such as magnesium stearate, a sweetening agent such as sucrose or saccharin, and a flavoring agent such as peppermint, methyl salicylate, or a fruit flavor.

The pharmaceutical composition of the present invention is administered in a pharmaceutically effective amount. In the present invention, 'pharmaceutically effective amount' refers to an amount sufficient to treat diseases at a reasonable benefit/risk ratio applicable to medical treatment, and an effective dosage level may be determined according to factors including types of diseases of patients, the severity of a disease, the activity of drugs, sensitivity to drugs, administration time, administration route, excretion rate, treatment period, and simultaneously used drugs, and other factors well known in the medical field. The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects in consideration of all the aforementioned factors, and this amount may be easily determined by a person skilled in the art.

### [Advantageous Effects]

A conjugate including an antibody for treating cancer, a linker, and a block copolymer including PEO and PPO, and a conjugate further including a low molecular weight compound have an excellent cancer cell targeting ability and can efficiently kill cancer cells by increasing the half-life of the antibody, and thus can be usefully used for treating cancer.

### [Description of Drawings]

FIG. 1 shows the results of confirming the ¹H-NMR spectrum of maleimide-Pluronic F68 (Mal-PF68) according to an exemplary embodiment of the present invention.
FIG. 2 shows the results of confirming the ¹H-NMR spectrum of maleimide-Pluronic F127 (Mal-PF127) according to an exemplary embodiment of the present invention.
FIG. 3 shows the results of confirming the ¹H-NMR spectrum of succinyl-Pluronic F68 (Suc-PF68) according to an exemplary embodiment of the present invention.
FIG. 4 shows the results of confirming the ¹H-NMR spectrum of succinyl-Pluronic F127 (Suc-PF127) according to an exemplary embodiment of the present invention.
FIG. 5 shows the results of confirming the ¹H-NMR spectrum of maleimide-polyethylene glycol 2k according to an exemplary embodiment of the present invention.
FIG. 6 shows the results of confirming the ¹H-NMR spectrum of maleimide-polyethylene glycol 6k according to an exemplary embodiment of the present invention.
FIG. 7 shows the results of confirming the molecular weight of cetuximab-maleimide-Pluronic F68 (CTX-Mal-PF68) by MALDI-TOF/MS spectrum.
FIG. 8A shows the results of confirming the molecular weight of trastuzumab-maleimide-Pluronic F68 (TRA-Mal-PF68) by MALDI-TOF/MS spectrum.
FIG. 8B shows the results of confirming the molecular weight of trastuzumab-succinyl-Pluronic F68 (TRA-Suc-PF68) by MALDI-TOF/MS spectrum.
FIG. 9A shows the results of confirming the molecular weight of avelumab-maleimide-Pluronic F68 (AVE-Mal-PF68) by MALDI-TOF/MS spectrum.
FIG. 9B shows the results of confirming the molecular weight of avelumab-succinyl-Pluronic F68 (AVE-Suc-PF68) by MALDI-TOF/MS spectrum.
FIG. 10 shows the results of confirming the molecular weight of ramucirumab-maleimide-Pluronic F68 (RAM-Mal-PF68) by MALDI-TOF/MS spectrum.
FIG. 11A shows the results of measuring the circular dichroism of a cetuximab-maleimide-Pluronic F68/F127 conjugate (CTX-Mal-PF68/PF127).
FIG. 11B shows the results of measuring the circular dichroism of a trastuzumab-maleimide-Pluronic F68/F127 conjugate (TRA-Mal-PF68/PF127) (B).
FIG. 11C shows the results of measuring the circular dichroism of an avelumab-maleimide-Pluronic F68/F127 conjugate (AVE-Mal-PF68/PF127).
FIG. 11D shows the results of measuring the circular dichroism of a ramucirumab-maleimide-Pluronic F68/F127 conjugate (RAM-Mal-PF68/PF127).
FIGS. 12A to 12D show the results of confirming cytotoxicity after treating a normal cell line in which an epidermal growth factor receptor (EGFR) is not expressed with a maleimide-Pluronic conjugate, a maleimide-polyethylene glycol conjugate, a cetuximab-maleimide-Pluronic conjugate or a cetuximab-maleimide-polyethylene glycol conjugate.
FIGS. 13A and 13B show the results of confirming cytotoxicity after treating an ovarian cancer cell line expressing an EGFR with a maleimide-Pluronic conjugate, cetuximab, or a cetuximab-maleimide-Pluronic conjugate.
FIGS. 14A to 14D show the results of confirming cytotoxicity after treating a normal cell line in which human epidermal growth factor receptor 2 (HER2) is not expressed with a maleimide-Pluronic conjugate, a maleimide-polyethylene glycol conjugate, a trastuzumab-maleimide-Pluronic conjugate or a trastuzumab-maleimide-polyethylene glycol conjugate.
FIGS. 15A to 15F show the results of confirming cytotoxicity after treating a breast cancer cell line expressing HER2 with a maleimide-Pluronic conjugate and a trastuzumab-maleimide-Pluronic conjugate.
FIGS. 16A to 16D show the results of confirming cytotoxicity after treating a normal cell line in which programmed death-ligand 1 (PD-L1) is not expressed with a maleimide-Pluronic conjugate, a maleimide-polyethylene glycol conjugate, an avelumab-maleimide-Pluronic conjugate or an avelumab-maleimide-polyethylene glycol conjugate.
FIGS. 17A to 17F show the results of confirming cytotoxicity after treating a cancer cell line expressing PD-L1 with a maleimide-Pluronic conjugate, avelumab, or an avelumab-maleimide-Pluronic conjugate.
FIGS. 18A to 18D show the results of confirming cytotoxicity after treating a normal cell line in which vascular endothelial growth factor receptor 2 (VEGFR2) is not expressed with a maleimide-Pluronic conjugate, a maleimide-polyethylene glycol conjugate, a ramucirumab-maleimide-Pluronic conjugate or a ramucirumab-maleimide-polyethylene glycol conjugate.
FIGS. 19A to 19D show the results of confirming cytotoxicity after treating a cancer cell line expressing PD-L1 with a maleimide-Pluronic conjugate, ramucirumab or a ramucirumab-maleimide-Pluronic conjugate.
FIGS. 20A and 20B show the results of confirming cytotoxicity after treating a breast cancer cell line expressing HER2 with a succinyl-Pluronic conjugate, a succinyl-polyethylene glycol conjugate, a trastuzumab-succinyl-Pluronic conjugate or a trastuzumab-succinyl-polyethylene glycol conjugate.
FIGS. 21A to 21D show the results of confirming cytotoxicity after treating a cancer cell line expressing PD-L1 with a succinyl-Pluronic conjugate, a succinyl-polyethylene glycol conjugate, an avelumab-succinyl-Pluronic conjugate or an avelumab-succinyl-polyethylene glycol conjugate.
FIG. 22 shows the results of confirming whether the conjugate is present in target cells according to the presence or absence of EGFR by flow cytometry after treating the cells with maleimide-Pluronic F68-chlorin e6, maleimide-polyethylene glycol 2K-chlorin e6, cetuximab-maleimide-Pluronic F68-chlorin e6 or a cetuximab-maleimide-polyethylene glycol 2k-chlorin e6 conjugate.
FIGS. 23A to 23C show the results of confirming whether the conjugate is present in target cells according to the presence or absence of EGFR expression by confocal laser scanning microscopy after treating the cells with maleimide-Pluronic F68-chlorin e6, maleimide-polyethylene glycol 2K-chlorin e6, cetuximab-maleimide-Pluronic F68-chlorin e6 or a cetuximab-maleimide-polyethylene glycol 2k-chlorin e6 conjugate.
FIGS. 24A and 24B show the results of confirming the *in vivo* behavior of the conjugate after injecting avelumab-chlorin e6, avelumab-maleimide-polyethylene glycol 2k-chlorin e6 or an avelumab-maleimide-Pluronic F68-chlorin e6 conjugate into mice.
FIGS. 25A and 25B shows the behavior of the conjugate in cancer tissue when cetuximab-maleimide-polyethylene glycol 2k-chlorin 36 or a cetuximab-maleimide-Pluronic F68-chlorin e6 conjugate is injected after the formation of cancer is induced in mice.
FIGS. 26A and 26B show the results of confirming the change in size of cancer tissue when cetuximab, cetuximab-maleimide-polyethylene glycol 2k, cetuximab-maleimide-polyethylene glycol 6k, cetuximab-maleimide-Pluronic F68 or a cetuximab-maleimide-Pluronic 127 conjugate is injected after the formation of cancer is induced in mice.
FIG. 27 shows the results of, after inducing the formation of cancer in mice, injecting cetuximab, cetuximab-maleimide-polyethylene glycol 2k, cetuximab-maleimide-polyethylene glycol 6k, cetuximab-maleimide-Pluronic F68 or a cetuximab-maleimide-Pluronic 127 conjugate, and confirming the change in size of cancer tissue in each mouse.
FIG. 28A shows the results of confirming the ability of the conjugate of cetuximab-maleimide-polyethylene glycol/Pluronic-chlorin e6 to produce singlet oxygen in an aqueous solution.
FIG. 28B shows the results of confirming the ability of the conjugate of trastuzumab-maleimide-polyethylene glycol/Pluronic-chlorin e6 (B) to produce singlet oxygen in an aqueous solution.

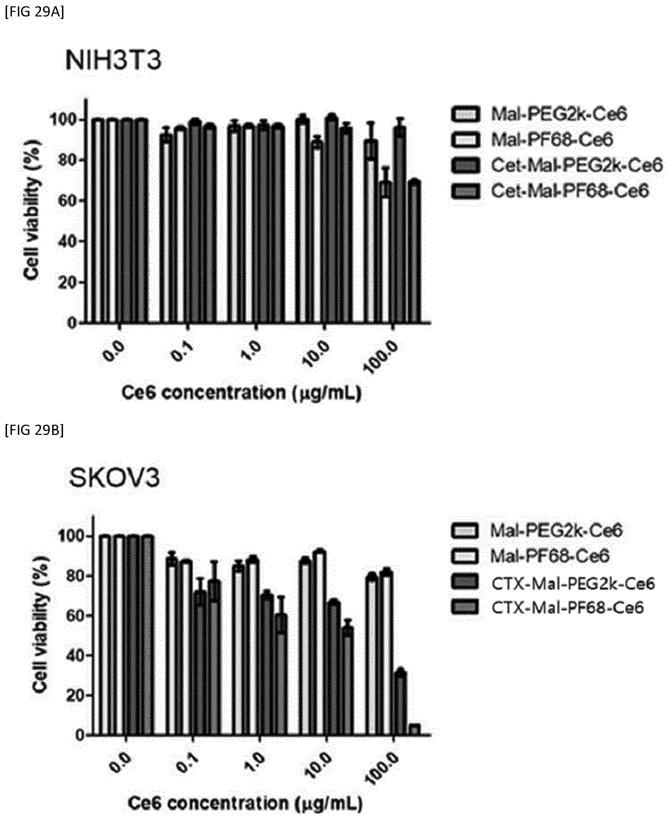

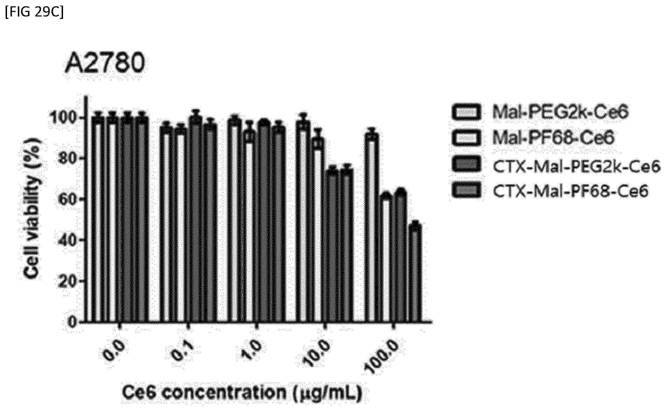

FIGS. 29A to 29C shows the results of confirming the cytotoxicity of a cetuximab-maleimide-polyethylene glycol/Pluronic-chlorin e6 conjugate per se in NIH-3T3 (A), SKOV-3 (B) and A-2780 (C) cells.
FIG. 30 shows the results of confirming the photodynamically mediated cytotoxicity of a cetuximab-maleimide-polyethylene glycol/Pluronic-chlorin e6 conjugate in NIH-3T3 (A), A-2780 (B) and SKOV-3 (C) cells.

### [Modes of the Invention]

Hereinafter, one or more specific exemplary embodiments will be described in more detail through examples. However, these examples are provided only for exemplarily explaining the one or more specific exemplary embodiments, and the scope of the present invention is not limited to these examples.

### Example 1: Preparation of linker-Pluronic conjugate

### 1-1. Maleimide-Pluronic F68 conjugate

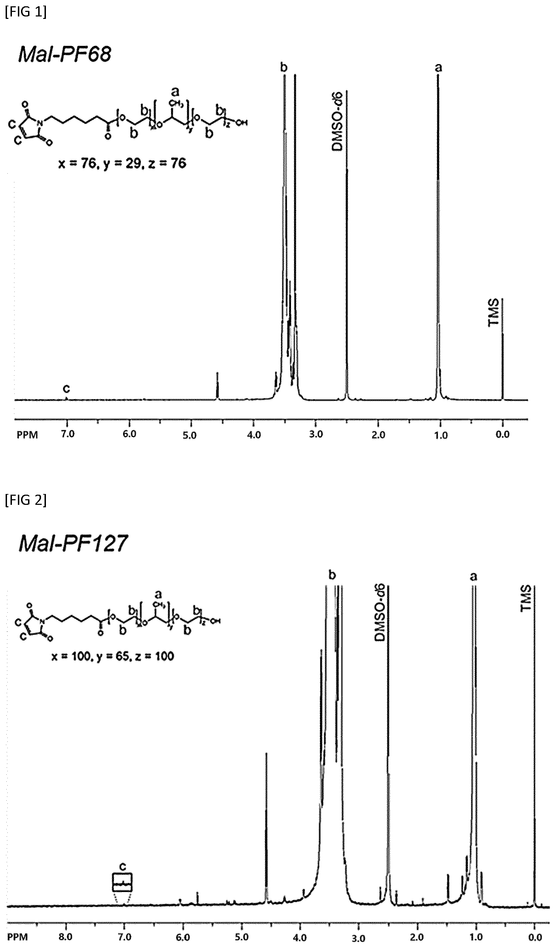

253 mg of 6-maleimidohexanoic acid (Mal), 297 mg of dicyclohexylcarbodiimide (DCC), and 317 mg of butylated hydroxy toluene (BHT) were dissolved in 3 ml of dimethylformamide (DMF). 1 g of a Pluronic 68 polymer was dissolved in 15 ml of dimethylformamide. After stirring each for 6 hours, a solution in which maleimidohexanoic acid was dissolved was added to the aqueous solution in which the Pluronic 68 polymer was dissolved, and the resulting mixture was stirred at room temperature for 48 hours. After the reaction was completed, the resulting product was crystallized in 40 ml of diethyl ether for purification. The supernatant other than the precipitate was discarded, and the recrystallization process in which diethyl ether was added again was performed three times in total to remove unreacted by-products. Thereafter, maleimide-Pluronic 68 powder (Mal-PF68) was obtained by drying the precipitate under reduced pressure. Synthesis results were confirmed by nuclear magnetic resonance spectrum (¹H-NMR) (FIG. 1).

### 1-2. Maleimide-Pluronic F127 conjugate

169 mg of 6-maleimidohexanoic acid, 198 mg of dicyclohexylcarbodiimide, and 212 mg of butylated hydroxytoluene were dissolved in 3 ml of dimethylformamide. 1 g of a Pluronic 127 polymer was dissolved in 15 ml of dimethylformamide. Each solution was stirred for 6 hours and subjected to the same process as in Example 1-1 to obtain maleimide-Pluronic 127 powder (Mal-PF127). Synthesis results were confirmed by nuclear magnetic resonance spectrum (¹H-NMR) (FIG. 2).

### 1-3. Preparation of succinyl-Pluronic F68 conjugate

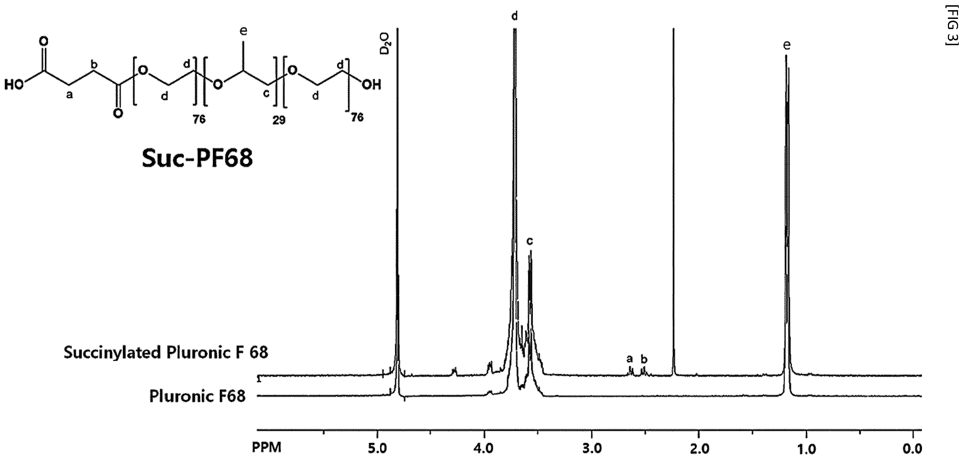

240 mg of succinic anhydride (Suc) and 260 mg of 4-dimethylaminopyridine (DMAP) were dissolved in 10 ml of dimethyl sulfoxide (DMSO). 1 g of Pluronic F68 was dissolved in 50 ml of dimethyl sulfoxide. Each solution was stirred for 6 hours, a solution in which succinic anhydride was dissolved was added to the aqueous solution in which the Pluronic 68 polymer was dissolved, and then the resulting mixture was stirred at room temperature for 24 hours. After the reaction was completed, the solution was dialyzed with a dialysis membrane (MWCO 3,500) for 2 days for purification. Thereafter, succinyl-Pluronic F68 dissolved in water was lyophilized to obtain a final powder (Suc-PF68). Synthesis results were confirmed by nuclear magnetic resonance spectrum (¹H-NMR) (FIG. 3).

### 1-4. Preparation of succinyl-Pluronic F127 conjugate

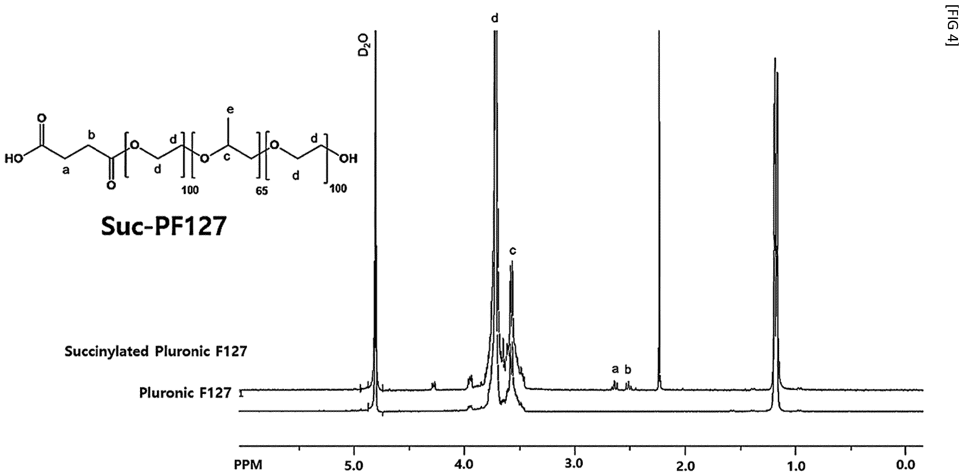

163 mg of succinic anhydride (Suc) and 179 mg of 4-dimethylaminopyridine (DMAP) were dissolved in 10 ml of dimethyl sulfoxide (DMSO). 1 g of Pluronic F127 was dissolved in 50 ml of dimethyl sulfoxide (DMSO). Thereafter, a final powder (Suc-PF127) was obtained through the same process as in Example 1-3. Synthesis results were confirmed by nuclear magnetic resonance spectrum (¹H-NMR) (FIG. 4).

### Example 2: Preparation of antibody-linker-Pluronic conjugate

### 2-1. Cetuximab-maleimide-Pluronic F68, Pluronic F127 conjugate

To remove salts, a cetuximab (CTX) injection formulation was passed through a PD10 column with 0.1 M PBS buffer (pH 7.4) as a mobile phase solvent to remove excipients or additives. 1 ml of the obtained material was collected to quantify the antibody by the bicinchoninic acid (BCA) method. Only 18 µl was taken from 2 mg of Traut's reagent and dispersed in 4 mg of a cetuximab aqueous solution to thiolate the amine group of the antibody for 1 hour. After 1 hour, it was purified with a PD10 column to remove the Traut's reagent. Thereafter, 1.13 mg of maleimide-Pluronic F68 (Mal-PF68) or 1.63 mg of maleimide-Pluronic F127 (Mal-PF127) was added to the aqueous solution in which 1 mg of cetuximab was dissolved, and the resulting mixture was stirred at room temperature for 4 hours. After the reaction was completed, the reaction product was centrifuged (14,000 g, 10 minutes) in an Amicon Ultra (15 ml, molecular weight cut-off size: 100,000 Da) tube to remove unreacted materials. Final products (CTX-Mal-PF68 and CTX-Mal-PF127) were stored under refrigeration.

### 2-2. Trastuzumab-maleimide-Pluronic F68, Pluronic F127 conjugate

Salts were removed from a trastuzumab (Tra) injection formulation in the same manner as in Example 2-1, the amine group of trastuzumab was thiolated, and then Traut's reagent was removed. Thereafter, 1.21 mg of maleimide-Pluronic F68 or 1.75 mg of maleimide-Pluronic F127 was added to the aqueous solution in which 1 mg of trastuzumab was dissolved, and the resulting mixture was stirred at room temperature for 4 hours. After the reaction was completed, the reaction product was centrifuged (14,000 g, 10 minutes) in an Amicon Ultra tube to remove unreacted materials. Final products (Tra-Mal-PF68 and Tra-Mal-PF127) were stored under refrigeration.

### 2-3. Avelumab-maleimide-Pluronic F68, Pluronic F127 conjugate

Salts were removed from an avelumab (AVE) injection formulation in the same manner as in Example 2-1, the amine group of avelumab was thiolated, and then Traut's reagent was removed. Thereafter, 1.2 mg of maleimide-Pluronic F68 or 1.72 mg of maleimide-Pluronic F127 was added to the aqueous solution in which 1 mg of avelumab was dissolved, and the resulting mixture was stirred at room temperature for 4 hours. After the reaction was completed, the reaction product was centrifuged (14,000 g, 10 minutes) in an Amicon Ultra tube to remove unreacted materials. Final products (AVE-Mal-PF68 and AVE-Mal-PF127) were stored under refrigeration.

### 2-4. Ramucirumab-maleimide-Pluronic F68, Pluronic F127 conjugate

Salts were removed from a ramucirumab injection formulation in the same manner as in Example 2-1, the amine group of ramucirumab was thiolated, and then Traut's reagent was removed. Thereafter, 1.2 mg of maleimide-Pluronic F68 or 1.72 mg of maleimide-Pluronic F127 was added to the aqueous solution in which 1 mg of ramucirumab was dissolved, and the resulting mixture was stirred at room temperature for 4 hours. After the reaction was completed, the reaction product was centrifuged (14,000 g, 10 minutes) in an Amicon Ultra tube to remove unreacted materials. Final products (RAM-Mal-PF68 and RAM-Mal-PF127) were stored under refrigeration.

### 2-5. Trastuzumab-succinyl-Pluronic F68, Pluronic F127 conjugate

Salts were removed from a trastuzumab injection formulation in the same manner as in Example 2-1, the antibody was quantified and 1 mg of the antibody was dissolved in a 0.5 M MES buffer. 0.031 mg of 1-3-dimethylaminopropyl-3-ethylcarbodiimide (EDC), 0.0188 mg of N-hydroxysuccinimide (NHS) and 1.2 mg of succinyl-Pluronic F68 or 1.9 mg of succinyl-Pluronic F127 were stirred in a 0.5 M MES buffer for 1 hour. This solution was added to the antibody-dissolved solution, and the resulting mixture was stirred at 4°C for 12 hours. After the reaction was completed, the reaction product was centrifuged (14,000 g, 10 minutes) in an Amicon Ultra tube to remove unreacted materials. Final products (Tra-Suc-PF68 and Tra-Suc-PF127) were stored under refrigeration.

### 2-6. Preparation of avelumab-succinyl-Pluronic F68, Pluronic F127 conjugate

Salts were removed from an avelumab injection formulation in the same manner as in Example 2-1, the antibody was quantified and 1 mg of the antibody was dissolved in a 0.5 M MES buffer. Thereafter, final products (AVE-Suc-PF68 and AVE-Suc-PF127) were prepared in the same manner as in Example 2-5, and stored under refrigeration.

### Example 3: Preparation of antibody-linker Pluronic-photosensitizer conjugate

### 3-1. Pluronic F68-chlorin e6 conjugate

110 mg of chlorin e6 (Ce6), 45 mg of dicylcohexylcarbodiimide (DCC) and 26 mg of 4-dimethylaminopyridine (DMAP) were dissolved in 5 ml of dichloromethane (DCM) in a 20 ml flask. 1 g of Pluronic F68 (PF68, 8400 g/mol) was dissolved in 10 ml of dichloromethane. After each solution was stirred for 6 hours, the two solutions were mixed, stirred at room temperature for 48 hours, and crystallized in 45 ml of diethyl ether. The supernatant other than the precipitate was discarded, the recrystallization process in which diethyl ether was added again was performed three times in total to remove unreacted by-products, and then the resulting product was dried under reduced pressure to obtain a powder. The powder was dissolved again in methanol at a concentration of 20 mg/ml and purified by open column chromatography to obtain synthesized Pluronic F68-chlorin e6 (PF68-Ce6).

### 3-2. Maleimide-Pluronic F68-chlorin e6 conjugate

23.2 mg of 6-maleimidohexanoic acid (Mal), 27.2 mg of dicyclohexylcarbodiimide (DCC), and 29.1 mg of butylated hydroxytoluene (BHT) were dissolved in 2 ml of dimethylformamide. 200 mg of the Pluronic F68-chlorin e6 (PF68-Ce6) conjugate obtained in Example 3-1 was dissolved in 3 ml of dimethylformamide. After each solution was stirred for 6 hours, a solution in which maleimidohexanoic acid was dissolved was added to the aqueous solution in which the Pluronic F68-chlorin e6 conjugate was dissolved, and the resulting mixture was stirred at room temperature for 48 hours. After the reaction was completed, the resulting product was crystallized in 45 ml of diethyl ether for purification. The supernatant other than the precipitate was discarded, the recrystallization process in which diethyl ether was added again was performed three times in total to remove unreacted by-products, and then the resulting product was dried under reduced pressure to obtain a final powder (Mal-PF68-Ce6).

### 3-3. Cetuximab-maleimide-Pluronic F68-chlorin e6 conjugate

Salts were removed from a cetuximab injection formulation in the same manner as in Example 2-1, the amine group of cetuximab was thiolated, and then Traut's reagent was removed. Thereafter, 3.63 mg of the maleimide-Pluronic F68-chlorin e6 (Mal-PF68-Ce6) prepared in Example 3-2 was added to an aqueous solution in which 1 mg of cetuximab was dissolved, and the resulting mixture was stirred at room temperature for 4 hours. After the reaction was completed, the reaction product was centrifuged (14,000 g, 10 minutes) in an Amicon Ultra tube to remove unreacted materials. A final product (CTX-Mal-PF68-Ce6) was stored under refrigeration.

### 3-4. Trastuzumab-maleimide-Pluronic F68-chlorin e6 conjugate

Salts were removed from a trastuzumab injection formulation in the same manner as in Example 2-1, the amine group of trastuzumab was thiolated, and then Traut's reagent was removed. Thereafter, 2.72 mg of the maleimide-Pluronic F68-chlorin e6 prepared in Example 3-2 was added to an aqueous solution in which 5 mg of trastuzumab was dissolved, and the resulting mixture was stirred at room temperature for 4 hours. After the reaction was completed, the reaction product was centrifuged (14,000 g, 10 minutes) in an Amicon Ultra tube to remove unreacted materials. A final product (Tra-Mal-PF68-Ce6) was stored under refrigeration.

### 3-5. Avelumab-maleimide-Pluronic F68-chlorin e6 conjugate

Salts were removed from an avelumab injection formulation in the same manner as in Example 2-1, the amine group of avelumab was thiolated, and then Traut's reagent was removed. Thereafter, 1.28 mg of the maleimide-Pluronic F68-chlorin e6 prepared in Example 3-2 was added to an aqueous solution in which 2 mg of avelumab was dissolved, and the resulting mixture was stirred at room temperature for 4 hours. After the reaction was completed, the reaction product was centrifuged (14,000 g, 10 minutes) in an Amicon Ultra tube to remove unreacted materials. A final product (AVE-Mal-PF68-Ce6) was stored under refrigeration.

### Comparative Example 1: Preparation of linker-polyethylene glycol conjugate

### 1-1. Maleimide-polyethylene glycol 2K conjugate

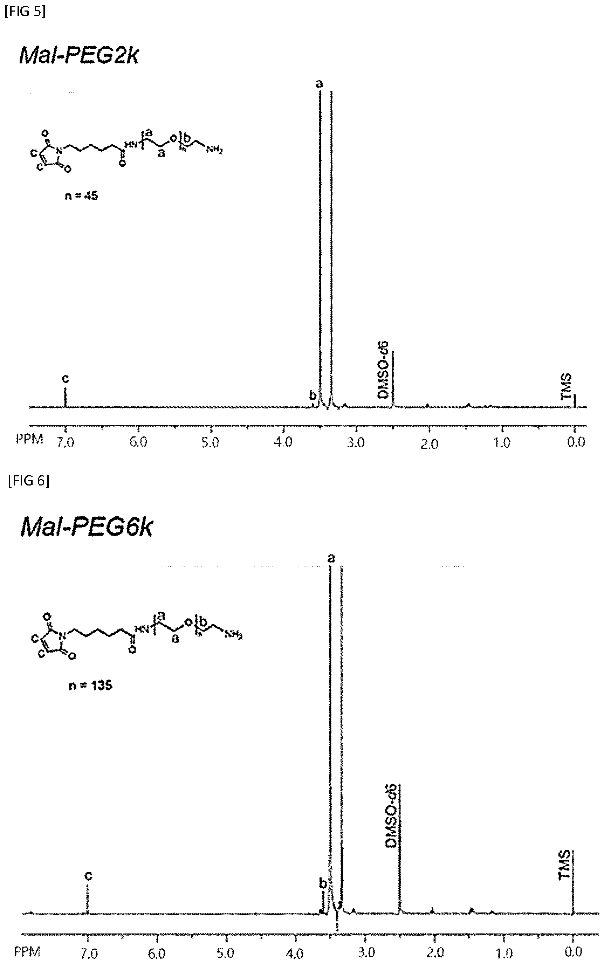

211 mg of 6-maleimidohexanoic acid, 247 mg of dicyclohexylcarbodiimide, and 138 mg of NHS were dissolved in 5 ml of dimethylformamide. 0.2 g of a polyethylene glycol 2K polymer was dissolved in 10 ml of dimethylformamide. After each solution was stirred for 4 hours, a solution in which maleimidohexanoic acid was dissolved was added to the aqueous solution in which the polyethylene glycol 2K polymer was dissolved, and the resulting mixture was stirred at room temperature for 24 hours. After the reaction was completed, the resulting product was crystallized with 40 ml of diethyl ether for purification. The supernatant other than the precipitate was discarded, and the recrystallization process in which diethyl ether was added again was performed three times in total to remove unreacted by-products. Thereafter, a maleimide-polyethylene glycol 2k powder (Mal-PEG2K) was obtained by drying the precipitate under reduced pressure. Synthesis results were confirmed by nuclear magnetic resonance spectrum (¹H-NMR) (FIG. 5).

### 1-2. Maleimide-polyethylene glycol 6K conjugate

70.3 mg of 6-maleimidohexanoic acid, 82.5 mg of dicyclohexylcarbodiimide, and 40.6 mg of NHS were dissolved in 5 ml of dimethylformamide. 0.2 g of a polyethylene glycol 6K polymer was dissolved in 10 ml of dimethylformamide. After each solution was stirred for 4 hours, a solution in which maleimidohexanoic acid was dissolved was added to the aqueous solution in which the polyethylene glycol 2K polymer was dissolved, and the resulting mixture was stirred at room temperature for 24 hours. After the reaction was completed, the resulting product was crystallized in 40 ml of diethyl ether for purification. The supernatant other than the precipitate was discarded, and the recrystallization process in which diethyl ether was added again was performed three times in total to remove unreacted by-products. Thereafter, a maleimide-polyethylene glycol 6K powder (Mal-PEG6K) was obtained by drying the precipitate under reduced pressure. Synthesis results were confirmed by nuclear magnetic resonance spectrum (¹H-NMR) (FIG. 6).

### Comparative Example 2: Preparation of antibody-linker-polyethylene glycol conjugate

### 2-1. Cetuximab-maleimide-polyethylene glycol 2K, polyethylene glycol 6K conjugates

Salts were removed from a cetuximab injection formulation in the same manner as in Example 2-1, the amine group of cetuximab was thiolated, and then Traut's reagent was removed. Thereafter, 0.44 mg of maleimide-polyethylene glycol 2K or 1.24 mg of maleimide-polyethylene glycol 6k was added to the aqueous solution in which 3 mg of cetuximab was dissolved, and the resulting mixture was stirred at room temperature for 4 hours. After the reaction was completed, the reaction product was centrifuged (14,000 g, 10 minutes) in an Amicon Ultra tube to remove unreacted materials. Final products (CTX-Mal-PEG2K and CTX-Mal-PEG6K) were stored under refrigeration.

### 2-2. Trastuzumab-maleimide-polyethylene glycol 2K, polyethylene glycol 6K conjugates

Salts were removed from a trastuzumab injection formulation in the same manner as in Example 2-1, the amine group of trastuzumab was thiolated, and then Traut's reagent was removed. Thereafter, 0.75 mg of maleimide-polyethylene glycol 2k or 2.1 mg of maleimide-polyethylene glycol 6k was added to the aqueous solution in which 5 mg of trastuzumab was dissolved, and the resulting mixture was stirred at room temperature for 4 hours. After the reaction was completed, the reaction product was centrifuged (14,000 g, 10 minutes) in an Amicon Ultra tube to remove unreacted materials. Final products (Tra-Mal-PEG2K and Tra-Mal-PEG6K) were stored under refrigeration.

### 2-3. Avelumab-maleimide-polyethylene glycol 2K, polyethylene glycol 6K conjugates

Salts were removed from an avelumab injection formulation in the same manner as in Example 2-1, the amine group of avelumab was thiolated, and then Traut's reagent was removed. Thereafter, 0.301 mg of maleimide-polyethylene glycol 2K or 0.845 mg of maleimide-polyethylene glycol 6K was added to the aqueous solution in which 2 mg of avelumab was dissolved, and the resulting mixture was stirred at room temperature for 4 hours. After the reaction was completed, the reaction product was centrifuged (14,000 g, 10 minutes) in an Amicon Ultra tube to remove unreacted materials. Final products (AVE-Mal-PEG2K and AVE-Mal-PEG6K) were stored under refrigeration.

### 2-4. Ramucirumab-maleimide-polyethylene glycol 2K, polyethylene glycol 6K conjugates

Salts were removed from a ramucirumab injection formulation in the same manner as in Example 2-1, the amine group of ramucirumab was thiolated, and then Traut's reagent was removed. Thereafter, 0.301 mg of maleimide-polyethylene glycol 2K or 0.845 mg of maleimide-polyethylene glycol 6K was added to the aqueous solution in which 2 mg of ramucirumab was dissolved, and the resulting mixture was stirred at room temperature for 4 hours. After the reaction was completed, the reaction product was centrifuged (14,000 g, 10 minutes) in an Amicon Ultra tube to remove unreacted materials. Final products (RAM-Mal-PEG2K and RAM-Mal-PEG6K) were stored under refrigeration.

### 2-5. Trastuzumab-succinyl-polyethylene glycol 2K, polyethylene glycol 5K conjugates

Salts were removed from a trastuzumab injection formulation in the same manner as in Example 2-1, the antibody was quantified and 1 mg of the antibody was dissolved in a 0.5 M MES buffer. 0.031 mg of EDC, 0.0188 mg of NHS and 0.27 mg of succinyl-polyethylene glycol 2K or 0.82 mg of succinyl-polyethylene glycol 5K were stirred in a 0.5 M MES buffer for 1 hour. This solution was added to the antibody-dissolved solution, and the resulting mixture was stirred at 4°C for 12 hours. After the reaction was completed, the reaction product was centrifuged (14,000 g, 10 minutes) in an Amicon Ultra tube to remove unreacted materials. Final products (Tra-Suc-PEG2K and Tra-Suc-PEG6k) were stored under refrigeration.

### 2-6. Avelumab-succinyl-polyethylene glycol 2K, polyethylene glycol 5K conjugates

Salts were removed from an avelumab injection formulation in the same manner as in Example 2-1, the antibody was quantified and 1 mg of the antibody was dissolved in a 0.5 M MES buffer. 0.031 mg of EDC, 0.0188 mg of NHS and 0.27 mg of succinyl-polyethylene glycol 2K or 0.82 mg of succinyl-polyethylene glycol 5K were stirred in a 0.5 M MES buffer for 1 hour. This solution was added to the antibody-dissolved solution, and the resulting mixture was stirred at 4°C for 12 hours. After the reaction was completed, the reaction product was centrifuged (14,000 g, 10 minutes) in an Amicon Ultra tube to remove unreacted materials. Final products (AVE-Suc-PEG2K and AVE-Suc-PEG6k) were stored under refrigeration.

### Comparative Example 3: Preparation of antibody-polyethylene glycol-photosensitizer conjugate

### 3-1. Cetuximab-maleimide-polyethylene glycol 2K-chlorin e6 conjugate

Salts were removed from a cetuximab injection formulation in the same manner as in Example 2-1, the amine group of cetuximab was thiolated, and then Traut's reagent was removed. Thereafter, 1.85 mg of maleimide-polyethylene glycol 2k-chlorin e6 was added to the aqueous solution in which 1 mg of cetuximab was dissolved, and the resulting mixture was stirred at room temperature for 4 hours. After the reaction was completed, the reaction product was centrifuged (14,000 g, 10 minutes) in an Amicon Ultra tube to remove unreacted materials. A final product (CTX-Mal-PEG2K-Ce6) was stored under refrigeration.

### 3-2. Trastuzumab-maleimide-polyethylene glycol 2K-chlorin e6 conjugate

Salts were removed from a trastuzumab injection formulation in the same manner as in Example 2-1, the amine group of trastuzumab was thiolated, and then Traut's reagent was removed. Thereafter, 0.949 mg of maleimide-polyethylene glycol 2k-chlorin e6 was added to the aqueous solution in which 5 mg of trastuzumab was dissolved, and the resulting mixture was stirred at room temperature for 4 hours. After the reaction was completed, the reaction product was centrifuged (14,000 g, 10 minutes) in an Amicon Ultra tube to remove unreacted materials. A final product (Tra-Mal-PEG2K-Ce6) was stored under refrigeration.

### 3-3. Avelumab-maleimide-polyethylene glycol 2K-chlorin e6 conjugate

Salts were removed from an avelumab injection formulation in the same manner as in Example 2-1, the amine group of avelumab was thiolated, and then Traut's reagent was removed. Thereafter, 0.764 mg of maleimide-polyethylene glycol 2K-chlorin e6 was added to the aqueous solution in which 2 mg of avelumab was dissolved, and the resulting mixture was stirred at room temperature for 4 hours. After the reaction was completed, the reaction product was centrifuged (14,000 g, 10 minutes) in an Amicon Ultra tube to remove unreacted materials. A final product (AVE-Mal-PEG2K-Ce6) was stored under refrigeration.

### Experimental Example 1: MALDI-TOF analysis of antibody-linker-Pluronic conjugate

In order to confirm whether the antibody-Pluronic F68 prepared in Examples 2-1 to 2-6 was conjugated, the molecular weight of each conjugate was measured using a MALDI-TOF analyzer.

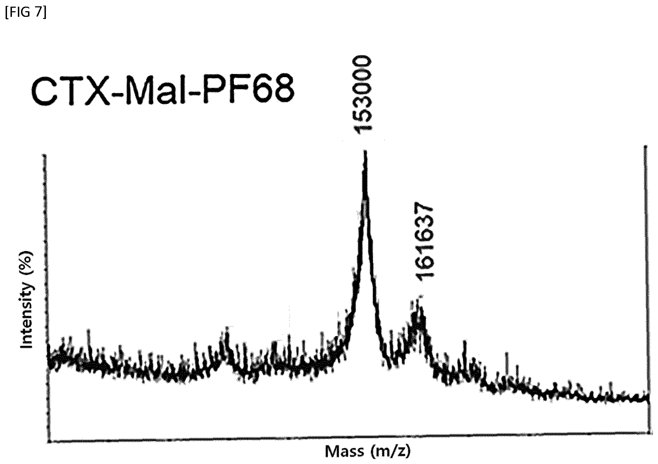

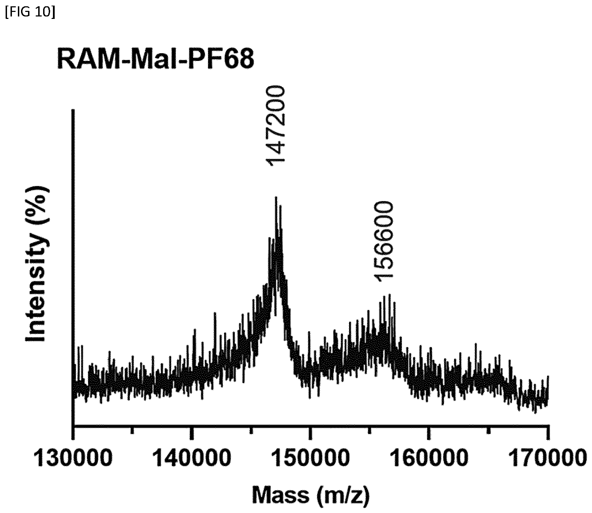

As a result, the molecular weights of cetuximab-maleimide-Pluronic F68, trastuzumab-maleimide-Pluronic F68, avelumab-maleimide-Pluronic F68 and ramucirumab-maleimide-Pluronic F68 were measured to be about 161, 157, 155, and 156 kDa, which were obtained by adding the molecular weights of cetuximab (152 kDa), trastuzumab (148 kDa), avelumab (147 kDa), ramucirumab (147 kDa) and a maleimide-Pluronic F68 conjugate (8611 g/mol), respectively (FIG. 7A, FIG. 8A, FIG. 9 and FIG. 10).

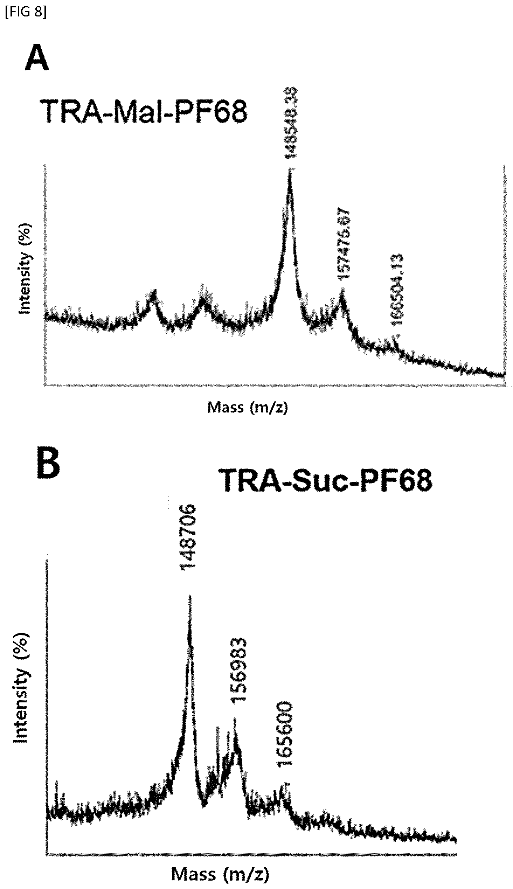

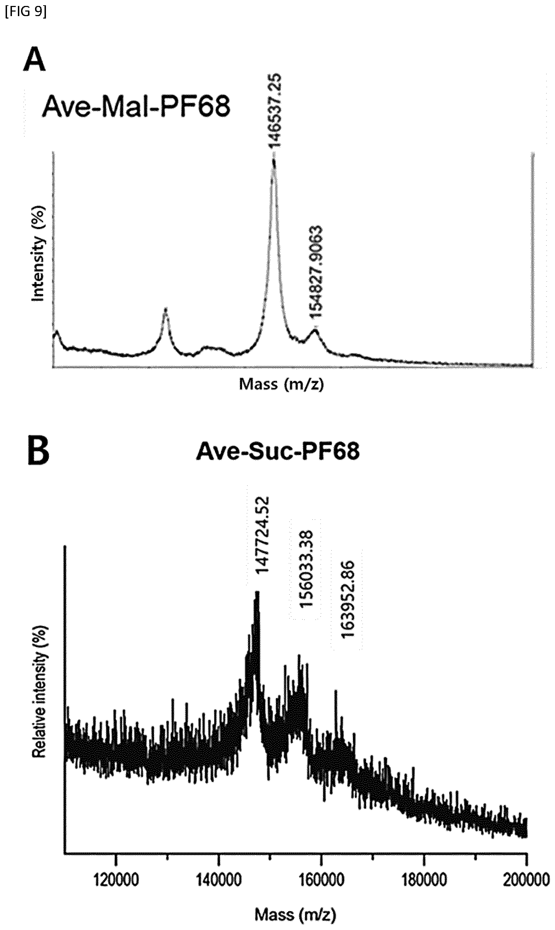

Further, the molecular weights of trastuzumab-succinyl-Pluronic F68 and avelumab-succinyl-Pluronic F68 were measured to be about 157 kDa and 156 kDa, which were obtained by adding the molecular weights of trastuzumab (148 kDa), avelumab (147 kDa) and a succinyl-Pluronic F68 conjugate (9033 g/mol), respectively (FIG. 8B and FIG. 9B).

From the results in FIGS. 7 to 10, it was confirmed that antibody-based Pluronic conjugates were successfully synthesized.

### Experimental Example 2: Evaluation of structural stability of antibody-linker-Pluronic conjugates

In order to confirm the structural stability of the antibody-maleimide-Pluronic F68 and antibody-maleimide-Pluronic F127 conjugates prepared in Examples 2-1 to 2-4 through the secondary structures thereof, circular dichroism was measured. Immunoglobulin G, which constitutes a monoclonal antibody, has an inherent secondary structure, and has a positive circular heterochromia at 202 nm and a negative value at 218 nm, due to this structure. Therefore, it is possible to indirectly confirm whether the antibody structure is maintained by measuring the circular dichroism.

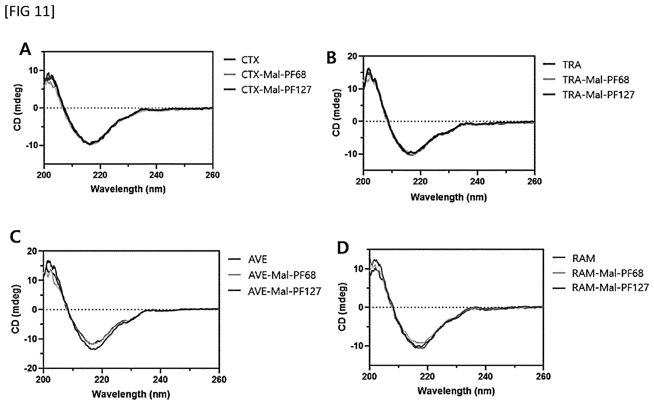

As a result of the measurement, it could be confirmed that the secondary structure of the monoclonal antibody which is an original material was well maintained in the antibody-maleimide-Pluronic conjugate (FIG. 11).

### Experimental Example 3: Confirmation of cancer cell-killing efficacy of antibody-linker-Pluronic conjugate

In order to compare the effects of the antibody-linker-Pluronic F68 and F127 conjugates prepared in Examples 2-1 to 2-6 with the effects of the antibody alone, the autonomous killing ability was confirmed in cancer cells with different expression levels of epidermal growth factor receptors. As comparative groups (Comparative Examples 2-1 to 2-6), an antibody-polyethylene glycol 2K, 5K or 6K conjugate having only a PEO block was used.

### 3-1. Cetuximab-maleimide-Pluronic F68, Pluronic F127 conjugates

L929 and NIH3T3 normal cells, which do not express an epidermal growth factor receptor (EGFR), and SKOV3 cells, which are an ovarian cell line expressing the EGFR, were cultured, and the cells were treated with cetuximab (CTX), cetuximab-maleimide-Pluronic F68 (CTX-Mal-PF68), or cetuximab-maleimide-Pluronic F127 (CTX-Mal-PF127) for 4 hours. Thereafter, the cells were washed with a buffer solution, and further cultured for 1 day by adding a clean medium thereto.

The cultured cells were treated with MTT reagent and further cultured for 3 hours, then the culture solution and the MTT reagent were both removed, and dimethyl sulfoxide was added to dissolve formazan formed in the cells. Thereafter, absorbance was measured at 570 nm to compare the amounts of formazan formed and analyze the viability and cytotoxicity of cancer cells.

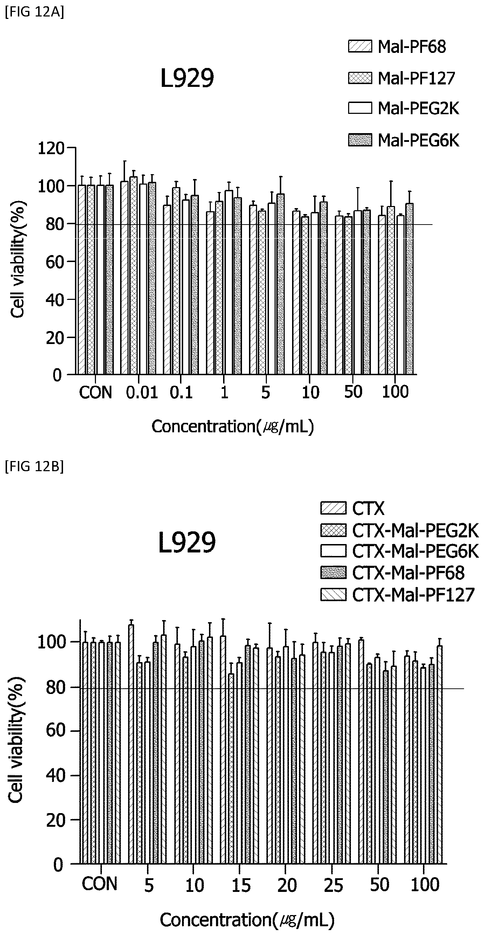

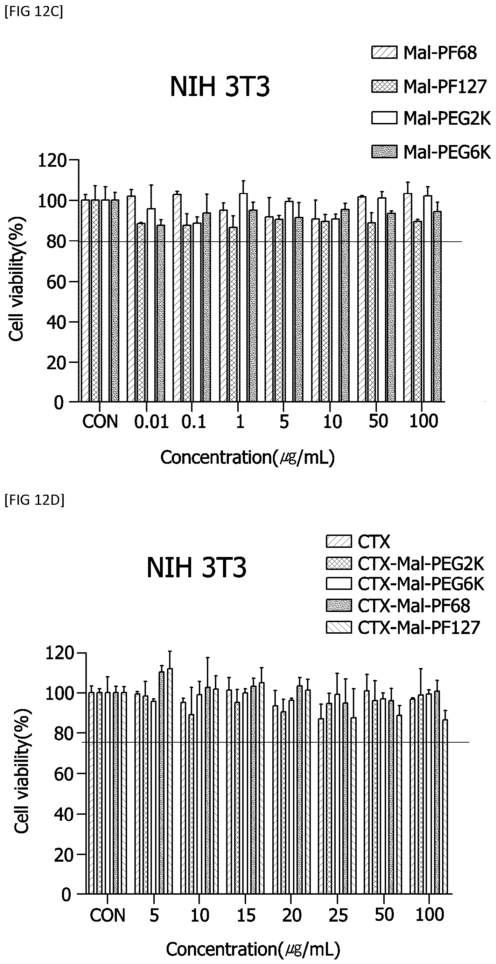

As a result of analysis, it was confirmed that in L929 and NIH3T3 cells, which are normal cells that do not express the EGFR, maleimide-Pluronic conjugates (Mal-PF68 and Mal-PF127), maleimide-polyethylene glycol conjugates (Mal-PEG2K and Mal-PEG6K), cetuximab-maleimide-Pluronic conjugates (CTX-Mal-PF68/PF127) and cetuximab-maleimide-polyethylene glycol conjugates (CTX-Mal-PEG2K/PEG6K) per se were non-cytotoxic (FIGS. 12A to 12D).

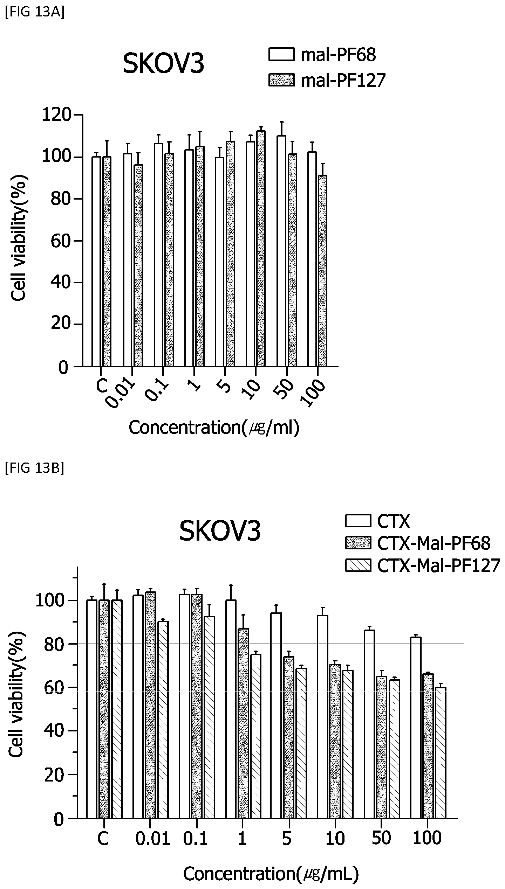

Maleimide-Pluronic conjugates (Mal-PF68/PF127) were non-cytotoxic even in an ovarian cancer cell line expressing EGFR. However, cetuximab-maleimide-Pluronic F68 (CTX-Mal-PF68) or cetuximab-maleimide-Pluronic F127 (CTX-Mal-PF127) treatment groups had more cell apoptosis than cetuximab antibody (CTX) treatment groups (FIGS. 13A and 13B).

Through the above results, it can be seen that more effective cancer treatment is possible when using cetuximab-maleimide-Pluronic F68 or cetuximab-maleimide-Pluronic F127 than cetuximab antibody alone.

### 3-2. Trastuzumab-maleimide-Pluronic F68, Pluronic F127 conjugates

L929 and NIH3T3 normal cells which do not express human epidermal growth factor receptor 2 (HER2) and breast cancer cell lines expressing HER2 (degree of HER2 expression: MDA-MB-231 < MCF-7 < SK-BR3) were cultured, and then treated with different concentrations of trastuzumab-maleimide-Pluronic conjugates. Thereafter, the viability and cytotoxicity of cells were analyzed in the same manner as in Experimental Example 2-1.

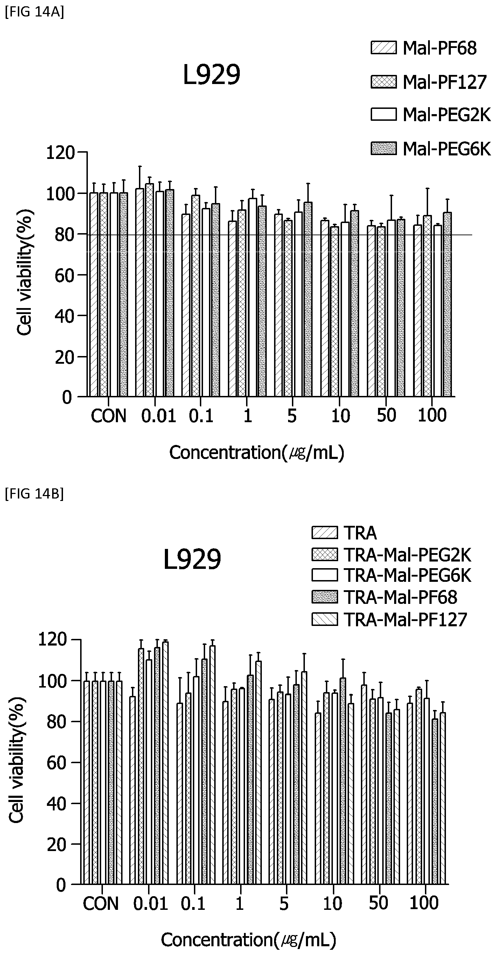

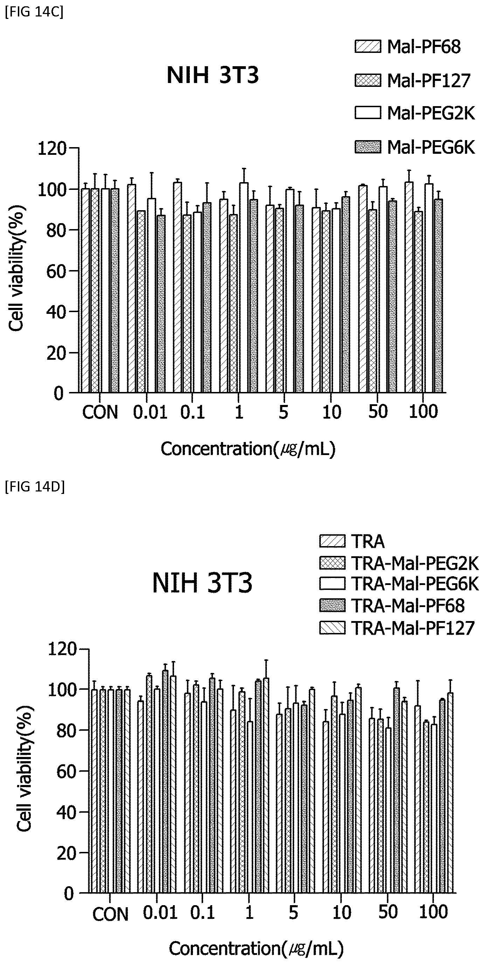

As a result, it was confirmed that in L929 and NIH3T3 cells, which do not express the EGFR, all of the maleimide-Pluronic conjugates (Mal-PF68 and Mal-PF127), maleimide-polyethylene glycol conjugates (Mal-PEG2K and Mal-PEG6K), trastuzumab-maleimide-polyethylene glycol (Tra-Mal-PEG2K and Tra-Mal-PEG6K) and trastuzumab-maleimide-polyethylene glycol conjugates (Tra-Mal-PF68 and Tra-Mal-PF127) were non-cytotoxic (FIGS. 14A to 14D).

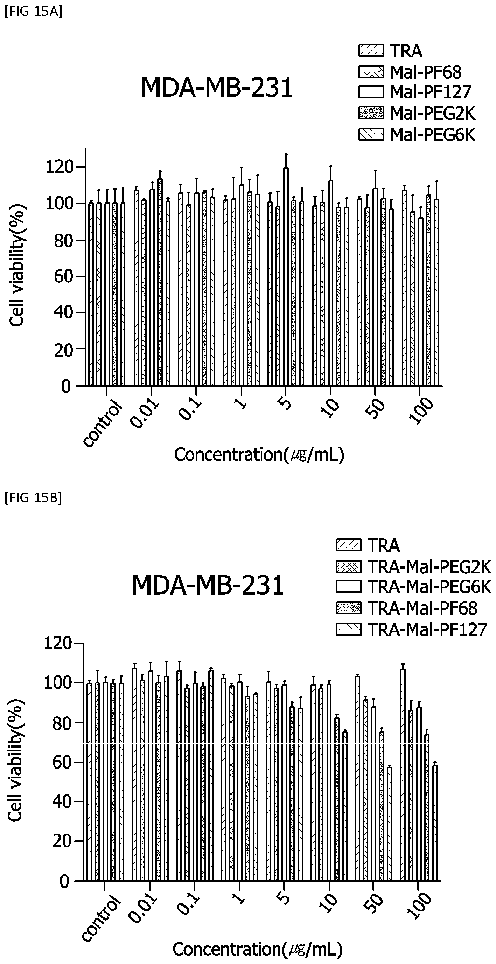

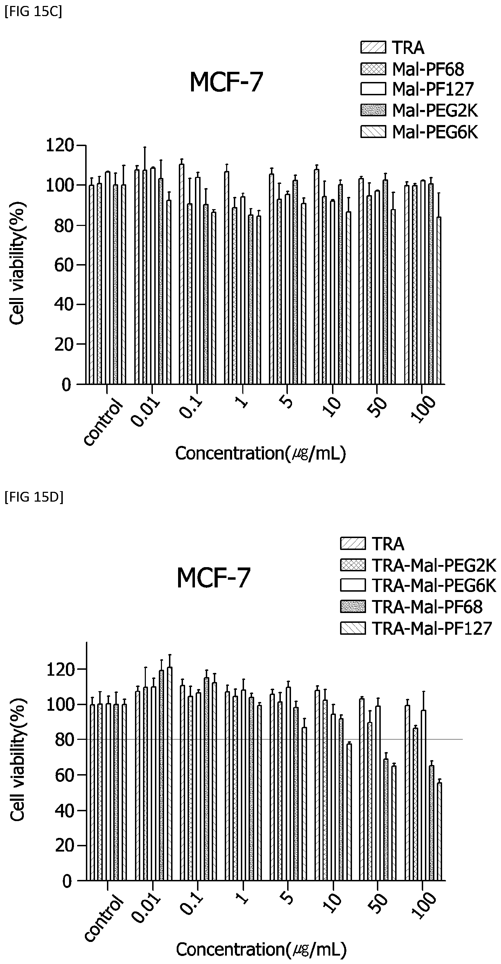

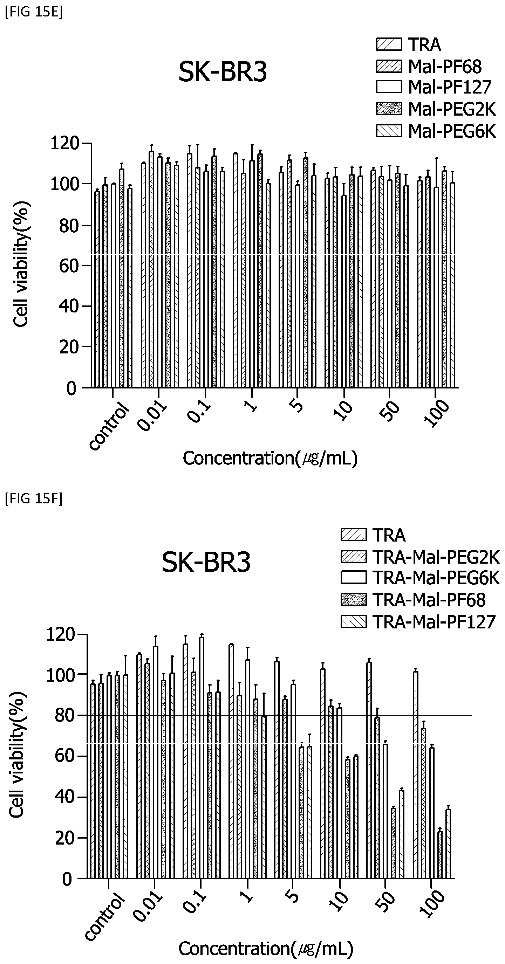

In addition, maleimide-Pluronic (Mal-PF68 and Mal-PF127) and maleimide-polyethylene glycol (Mal-PEG2K and Mal-PEG6K) conjugates per se were not toxic even in breast cancer cell lines expressing HER2 (FIGS. 15A, 15C and 15E). However, since it was found that cytotoxicity was increased in the trastuzumab-maleimide-Pluronic conjugate treatment group compared to the trastuzumab antibody alone or the trastuzumab-maleimide-polyethylene glycol conjugate treatment group, it was confirmed that an effective cancer treatment is possible (FIGS. 15B, 15D and 15F). Furthermore, an antigen-specific therapeutic effect was confirmed through an increase in toxicity of the trastuzumab-maleimide-Pluronic conjugate in proportion to the HER2 expression rate of the cell line.

### 3-3. Avelumab-maleimide-Pluronic F68, Pluronic F127 conjugates

L929 and NIH3T3 cell lines, which are normal cells that do not express programmed death-ligand 1 (PD-L1), and melanoma, colorectal cancer, and lung cancer cell lines expressing PD-L1 (B16F10, HCTE116, and A549, respectively) were cultured, and then treated with an avelumab-maleimide-Pluronic conjugate. Thereafter, the viability and cytotoxicity of cells were analyzed in the same manner as in Experimental Example 2-1.

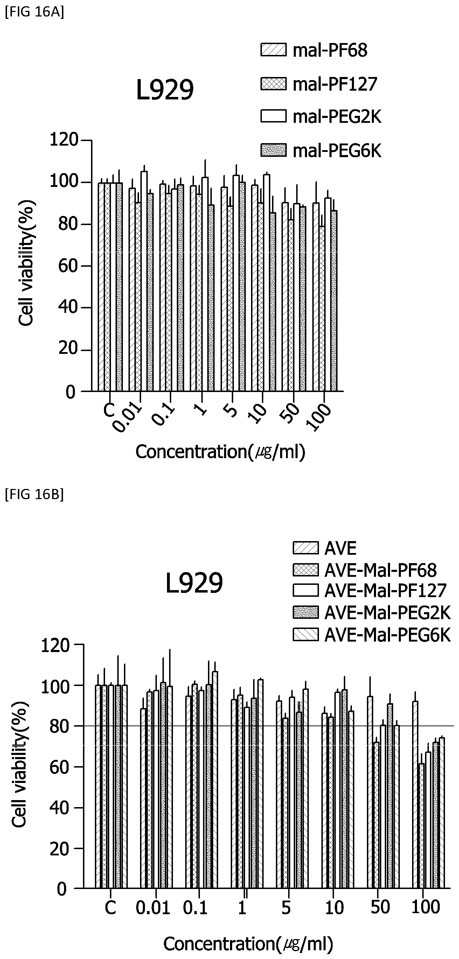

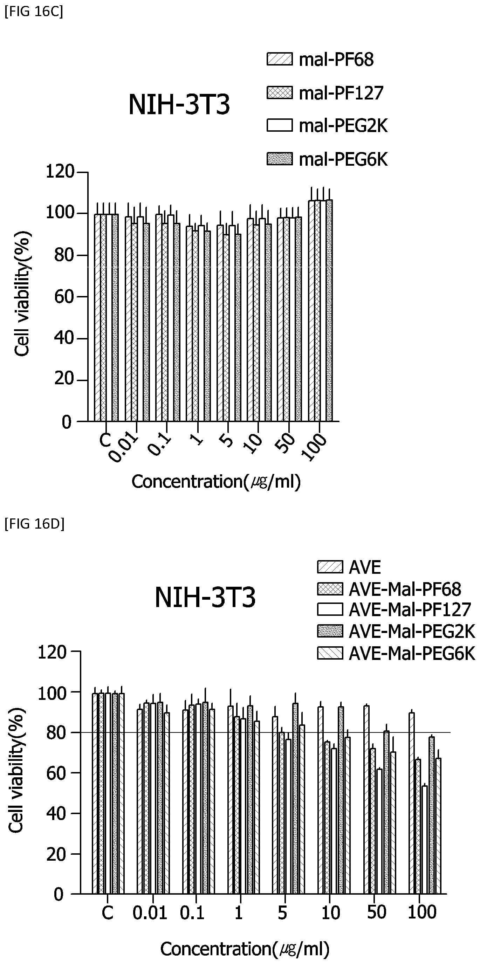

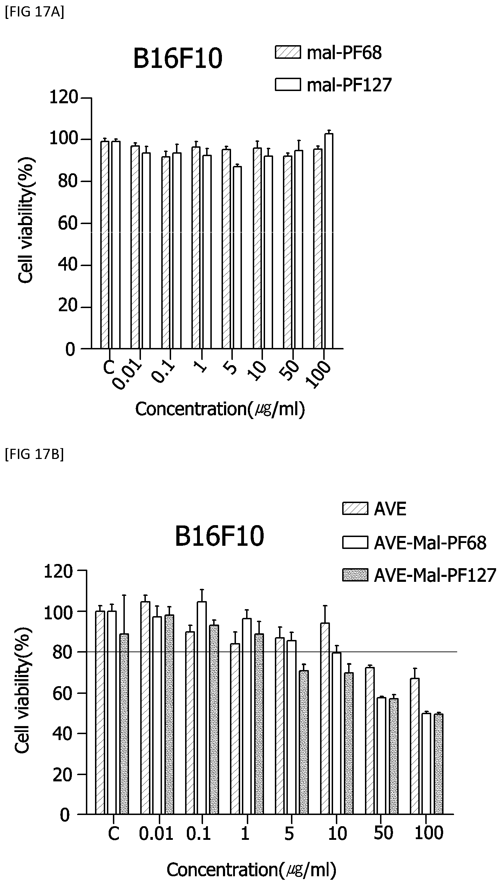

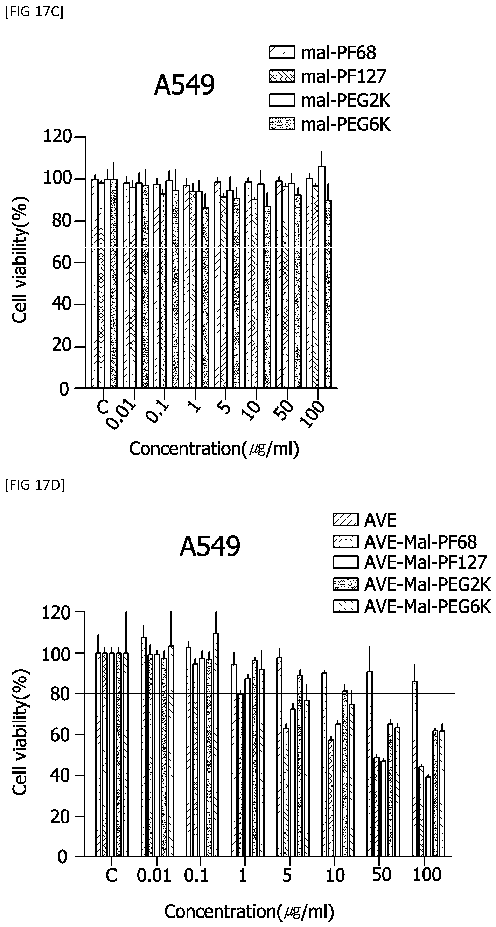

Maleimide-Pluronic (Mal-PF68 and Mal-PF127) or maleimide-polyethylene glycol conjugates (Mal-PEG2K and Mal-PEG6K) per se were non-toxic in L929 and NIH3T3 cells which do not express PD-L1 (FIGS. 16A and 16C). It was confirmed that all of the avelumab-maleimide-Pluronic (AVE-Mal-PF68 and AVE-Mal-PF127) or avelumab-maleimide-polyethylene glycol (AVE-Mal-PEG2K and AVE-Mal-PEG6K) conjugates were also non-toxic at a concentration less than 10 µl/ml (FIGS. 17B and 17D).

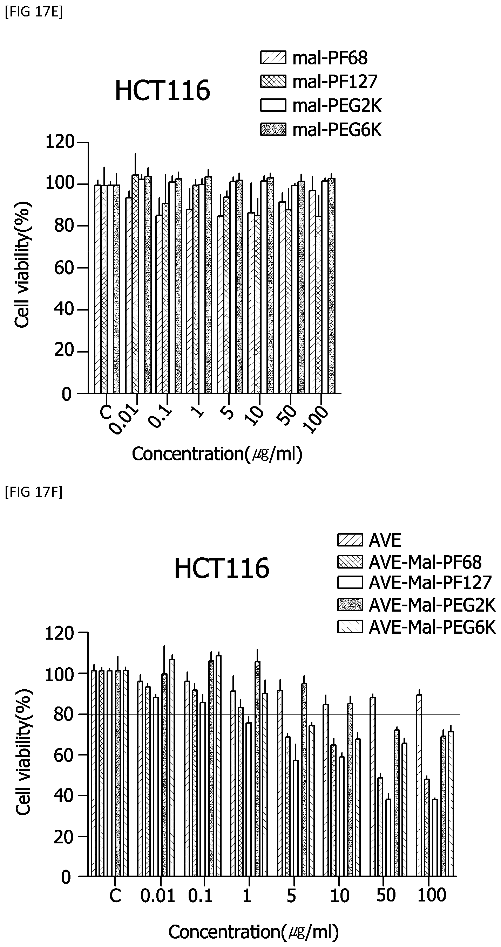

Further, maleimide-Pluronic (Mal-PF68 and Mal-PF127) and maleimide-polyethylene glycol (Mal-PEG2K and Mal-PEG6K) conjugates per se were non-toxic even in breast cancer cell lines expressing PD-L1 (FIGS. 17A, 17C and 17E). However, the avelumab-maleimide-Pluronic conjugate treatment group exhibited toxicity at a concentration of 5 µg/ml or less, showing a greater increase in cytotoxicity compared to the avelumab antibody alone (AVE) or avelumab-maleimide-polyethylene glycol treatment groups (FIGS. 17B, 17D and 17F), and through this, it was confirmed that an effective cancer treatment is possible.

### 3-4. Ramucirumab-maleimide-Pluronic F68, Pluronic F127 conjugates

L929 and NIH3T3 normal cells which do not express vascular endothelial growth factor receptor 2 (VEGFR2), a gastric cancer cell line (AGS), and non-small cell lung cancer (HCC15) were cultured, and then treated with different concentrations of ramucirumab-maleimide-Pluronic conjugates. Thereafter, the viability and cytotoxicity of cells were analyzed in the same manner as in Experimental Example 2-1.

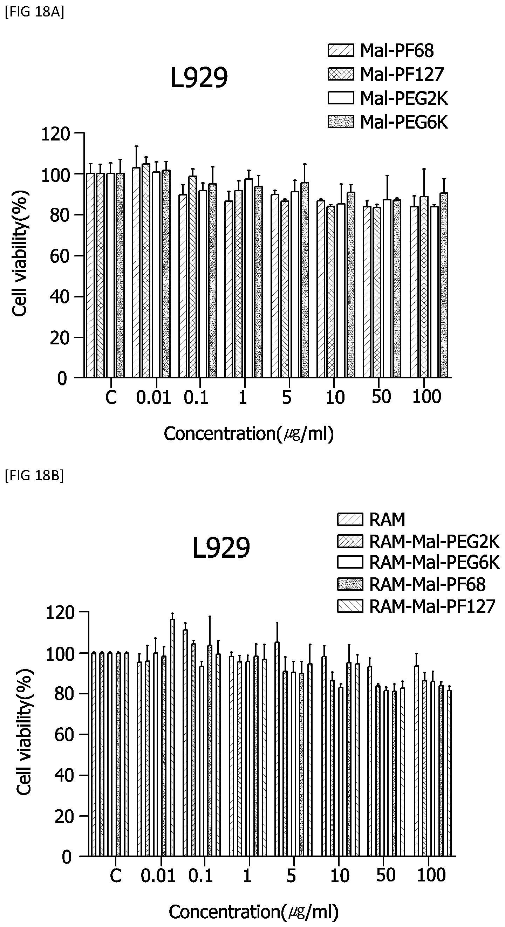

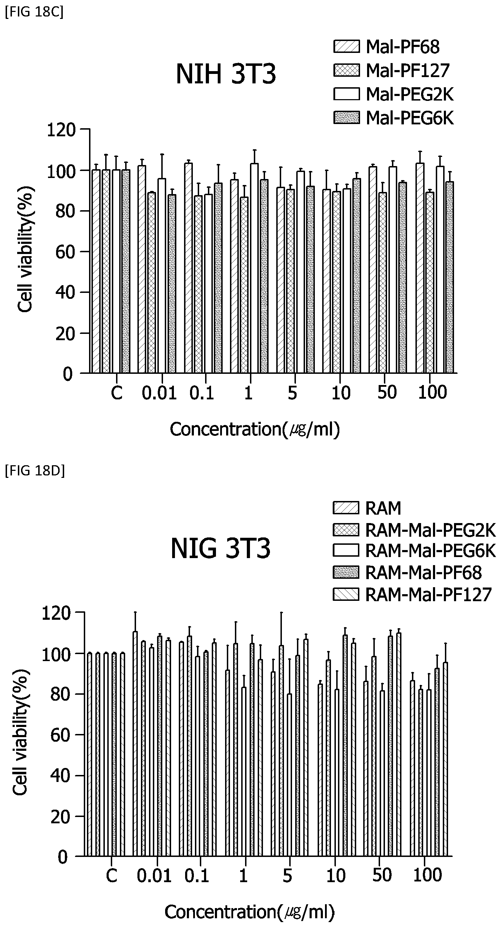

As a result of confirmation, it was confirmed that in normal cells which do not express VEGFR2, maleimide-Pluronic (Mal-PF68 and Mal-PF127) and maleimide-polyethylene glycol (Mal-PEG2K and Mal-PEG6K) conjugates were non-cytotoxic (FIGS. 18A and 18C), and ramucirumab-maleimide-Pluronic (RAM-Mal-PF68 and RAM-Mal-PF127) and ramucirumab-maleimide-polyethylene (RAM-Mal-PEG2K and RAM-Mal-PEG6K) conjugates were also non-toxic (FIGS. 18B and 18D).

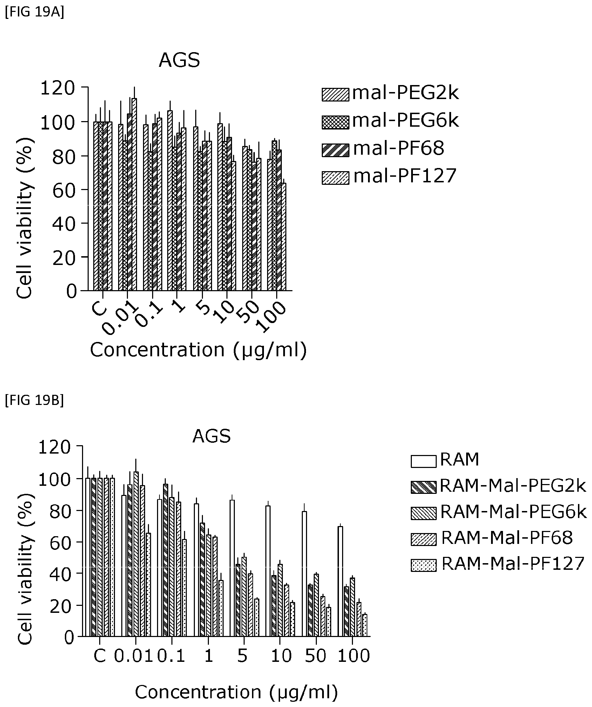

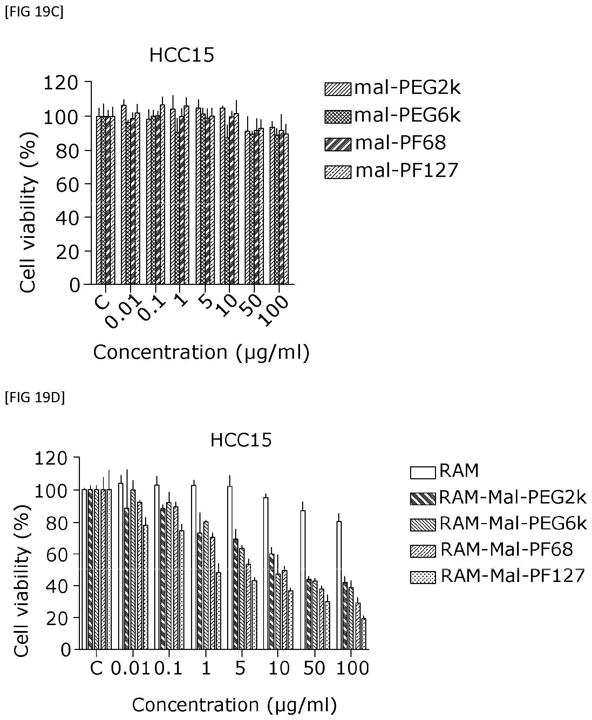

In addition, maleimide-Pluronic (Mal-PF68 and Mal-PF127) and maleimide-polyethylene glycol (Mal-PEG2K and Mal-PEG6K) conjugates were non-cytotoxic even in cancer cell lines expressing VEGFR2 (FIGS. 19A and 19C). However, cytotoxicity was further increased in the ramucirumab-maleimide-Pluronic conjugate treatment group compared to the ramucirumab antibody alone (RAM) or ramucirumab-maleimide-polyethylene glycol treatment groups (FIGS. 19B and 19D), and through this, it was confirmed that effective cancer treatment is possible.

### 3-5. Trastuzumab-succinyl-Pluronic conjugate

A breast cancer line expressing HER2 (SK-BR3) was cultured, and then treated with a trastuzumab-succinyl-Pluronic conjugate, and the viability and cytotoxicity of cells were analyzed in the same manner as in Experimental Example 2-1.

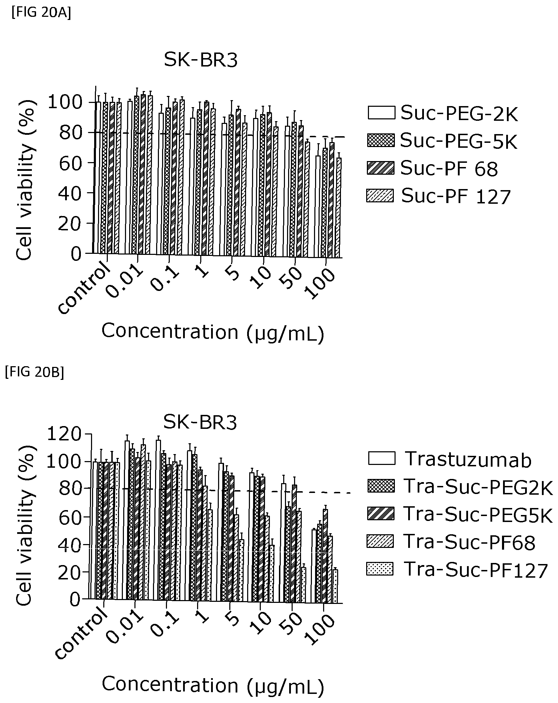

As a result, it was confirmed that in a breast cancer cell line SK-BR3 expressing HER2, neither succinyl-Pluronic conjugates (Suc-PF68 and Suc-PF127) nor succinyl-polyethylene glycol (Suc-PEG2K and Suc-PEG5K) conjugates are toxic at a concentration less than 50 µg/ml (FIG. 20A). In contrast, it could be seen that effective cancer treatment is possible by confirming that cytotoxicity was further increased in the trastuzumab-succinyl-Pluronic treatment group compared to the trastuzumab alone treatment group or trastuzumab-succinyl-polyethylene glycol conjugate treatment group (FIG. 20B).

### 3-6. Avelumab-succinyl-Pluronic conjugate

Lung cancer and colorectal cancer cell lines expressing PD-L1 (A549 and HCT116, respectively) were cultured, and then treated with an avelumab-succinyl-Pluronic conjugate. Thereafter, the viability and cytotoxicity of cells were analyzed in the same manner as in Experimental Example 2-1.

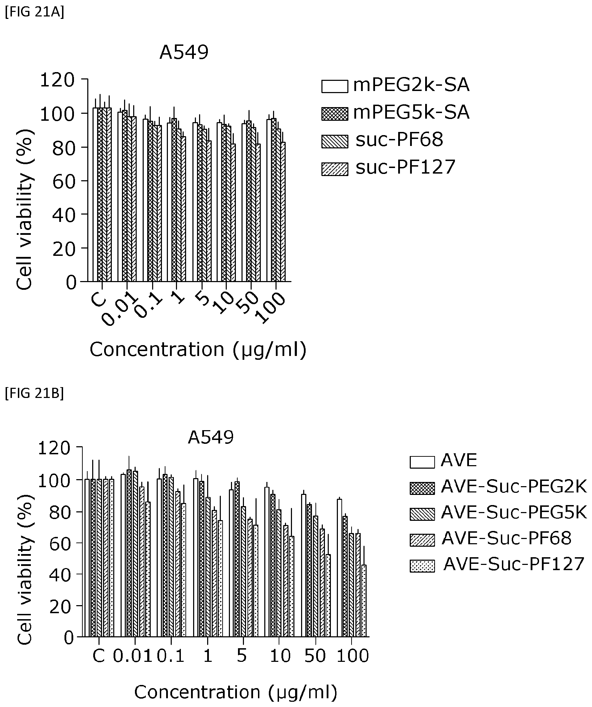

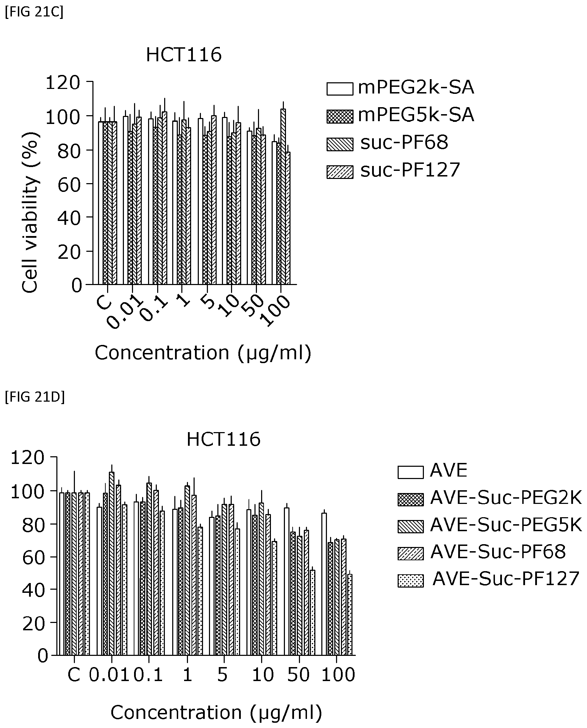

As a result, it was confirmed that succinyl-Pluronic (Suc-PF68 and Suc-PF127) and succinyl-polyethylene glycol (Suc-PEG2K and Suc-PEG5K) conjugates per se are non-cytotoxic in cancer cells expressing PD-L1 (FIGS. 21A and 21C). However, the avelumab-succinyl-Pluronic conjugate (AVE-Suc-PF68/PF127) treatment groups exhibited toxicity at a concentration of 5 µg/ml or less, showing a greater increase in cytotoxicity compared to the avelumab antibody alone (AVE) or avelumab-succinyl-polyethylene glycol (AVE-Suc-PEG2K/PEG5K) treatment groups (FIGS. 21B and 21D), and through this, it was confirmed that an effective cancer treatment is possible.

### Experimental Example 4: Quantitative evaluation of targeting ability of antibody-linker-Pluronic-photosensitizer conjugate according to expression degree of cancer cell receptor

The targeting ability of the cetuximab-maleimide-Pluronic F68-chlorin e6 conjugate according to cells with different levels of epidermal growth factor receptor (EGFR) expression was quantitatively confirmed compared to cetuximab-maleimide-polyethylene glycol 2k-chlorin e6, which is a comparative group.

After NIH-3T3 cells, which are normal cells and do not express EGFR, A2780 cells, which are cancer cells and have a low level of EGFR expression, and SKOV3 cells, which are cancer cells and have a high level of EGFR expression, were each cultured, each type of cell was treated with an antibody-linker-Pluronic-fluorescent material for 2 hours. Thereafter, the cells were washed with a buffer solution and the targeting ability to each type of cell was analyzed by a flow cytometer.

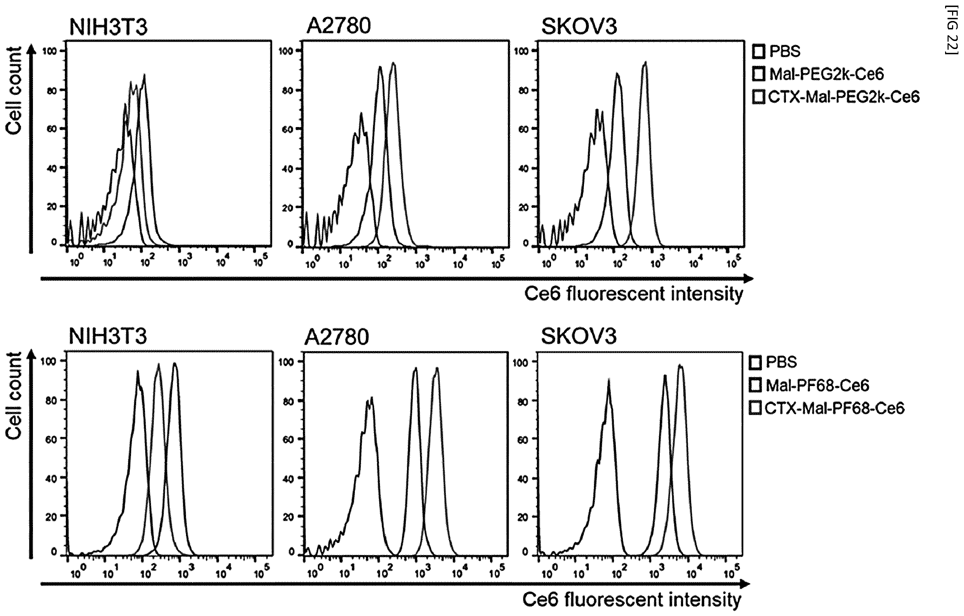

As a result, since NIH-3T3 cells do not express EGFR, and conjugates (maleimide-Pluronic F68-chlorin e6 and maleimide-polyethylene glycol 2K-chlorin e6) which are not linked to antibodies non-specifically flow into the cells, fluorescence signals were increased in the maleimide-Pluronic F68-chlorin e6 (Mal-PF68-Ce6) or maleimide-polyethylene glycol 2K-chlorin e6 (Mal-PEG2K-Ce6) treatment group compared to the cetuximab-maleimide-Pluronic F68-chlorin e6 (CTX-Mal-PF68-Ce6) or cetuximab-maleimide-polyethylene glycol 2k-chlorin e6 (CTX-Mal-PEG2K-Ce6) conjugate treatment group (FIG. 22).

In A2780 and SKOV3 cells expressing EGFR, the cetuximab-maleimide-Pluronic F68-chlorin e6 treatment group showed enhanced fluorescence compared to the cetuximab-maleimide-polyethylene glycol 2k-chlorin e6 treatment group, thus confirming that the targeting ability was enhanced (FIG. 22). Through this, it was confirmed that cetuximab-maleimide-Pluronic F68 further enhanced the targeting ability of the antibody compared to the polyethylene polymer.

From the above results, it was confirmed that the antibody-maleimide-Pluronic conjugate can more effectively target cancer cells having an epidermal growth factor receptor.

### Experimental Example 5: Confirmation of cell targeting of antibody-linker-Pluronic-photosensitizer conjugate according to expression degree of cancer cell receptor (fluorescence intensity)

The cell targeting of the cetuximab-maleimide-Pluronic F68-chlorin e6 according to cells with different levels of EGFR expression was visually confirmed by confocal laser scanning microscopy compared to cetuximab-maleimide-polyethylene glycol 2k-chlorin e6.

After NIH-3T3 cells, which are normal cells and do not express EGFR, A2780 cells, which are cancer cells and have a low level of EGFR expression, and SKOV3 cells, which are cancer cells and have a high level of EGFR expression, were each cultured, each type of cell was treated with an antibody-linker-Pluronic-fluorescent material for 2 hours. Thereafter, the treated cells were washed with a buffer solution and fixed with 4% paraformaldehyde.

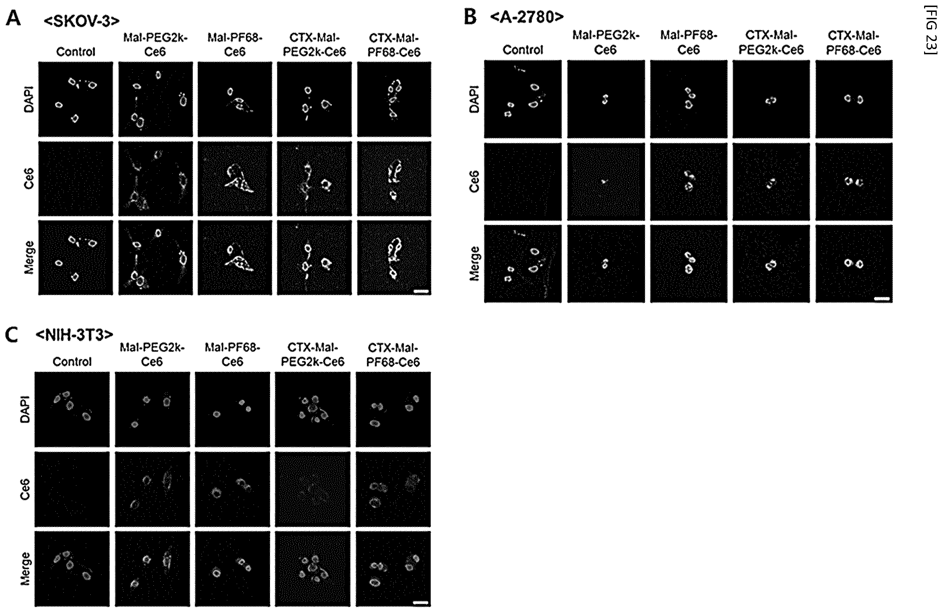

As a result, it was confirmed that in NIH-3T3 cells, which are normal cells, a fluorescence signal was scarcely detected both before and after the introduction of cetuximab (FIG. 23C), and in cancer cells, fluorescence signals differed significantly before and after antibody introduction in proportion to the expression level of EGFR. In particular, it could be clearly visually confirmed that SKOV3 cells with the highest EGFR expression level had increased fluorescence intensity after the introduction of cetuximab compared to A2780 cells. Further, it was confirmed that the fluorescence intensity was stronger in the cetuximab-maleimide-Pluronic F68-chlorin e6 (CTX-Mal-PF68-Ce6) treatment group compared to the cetuximab-maleimide-polyethylene glycol 2k-chlorin e6 (CTX-Mal-PEG2K-Ce6) treatment group (FIGS. 23A and 23B).

From the above results, it was confirmed that an antibody-based Pluronic polymer composition can more effectively target cancer cells having an epidermal growth factor receptor.

### Experimental Example 6: In vivo distribution of antibody-linker-Pluronic-photosensitizer conjugates

After male athymic nude mice (BALB/c nude mice, 5 weeks old) were intravenously injected with avelumab-maleimide-Pluronic F68-chlorin e6 (AVE-Mal-PF68-Ce6) at a concentration of 1.78 mg/kg based on chlorin e6, fluorescence images were acquired with a fluorescence labeled organism bioimaging (FOBI, NeoScience, Suwon, Korea) device for 144 hours to confirm the behavior of the conjugate. As comparison groups, avelumab-maleimide-polyethylene glycol 2k-chlorin e6 (AVE-Mal-Peg2K-Ce6) and avelumab-chlorin e6 (AVE-Ce6) conjugates were used.

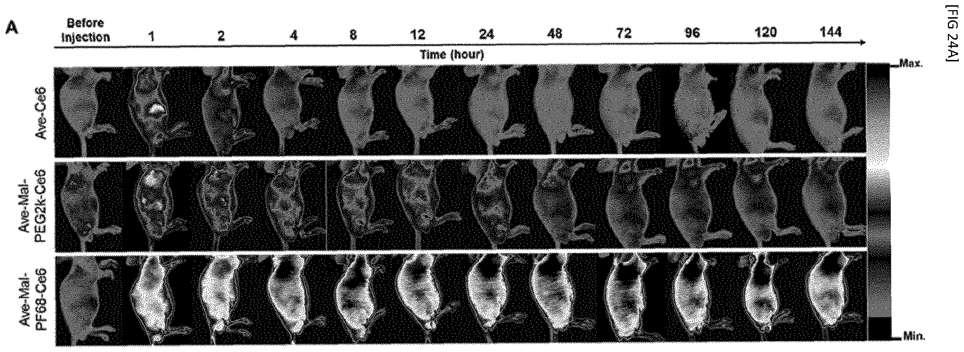

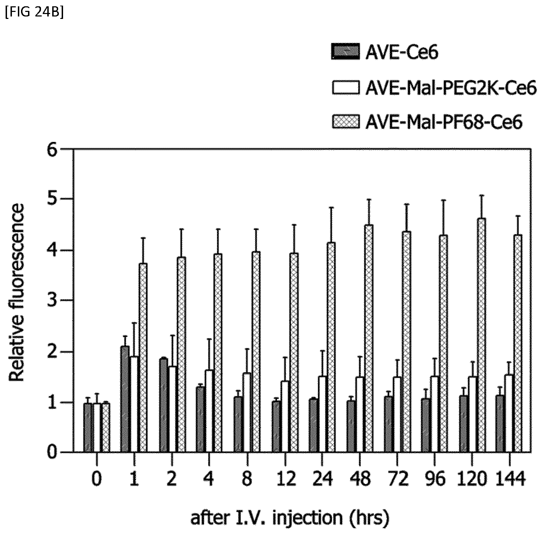

As a result of confirmation, stronger fluorescence could be confirmed for a significantly longer time in the avelumab-maleimide-Pluronic F68-chlorin e6 injection group compared to the avelumab-maleimide-polyethylene glycol 2k-chlorin e6 and avelumab-chlorin e6 injection groups (FIG. 24A). As a result of quantifying fluorescence signals according to each time period after representing fluorescence at 0 as 1 in order to express the fluorescence signal numerically, it was confirmed that at 144 hours, fluorescence observed in the avelumab-maleimide-Pluronic F68-chlorin e6 injection group was about 2.7 times that observed in the avelumab-chlorin e6 or avelumab-maleimide-polyethylene glycol 2k-chlorin e6 injection group (FIG. 24B).

From the above results, it was confirmed that the antibody-maleimide-Pluronic conjugate remained in the body for a longer time due to increased half-life.

### Experimental Example 7: Evaluation of in vivo cancer targeting ability of antibody-linker-Pluronic-photosensitizer conjugate

Male athymic nude mice (BALB/c nude mice, 5 weeks old) were subcutaneously injected with 1x10⁷ ASPC-1 cancer cells, and then when the size of cancer reached 80 mm³ after 15 days, a cetuximab-maleimide-Pluronic F68-chlorin e6 conjugate (CTX-Mal-PF68-Ce6) was intravenously injected at a concentration of 0.5 mg/kg based on chlorin e6. Thereafter, the fluorescence images of cancer tissue were acquired with a fluorescence labeled organism bioimaging (FOBI, NeoScience, Suwon, Korea) device for 120 hours to confirm the behavior of the conjugate.

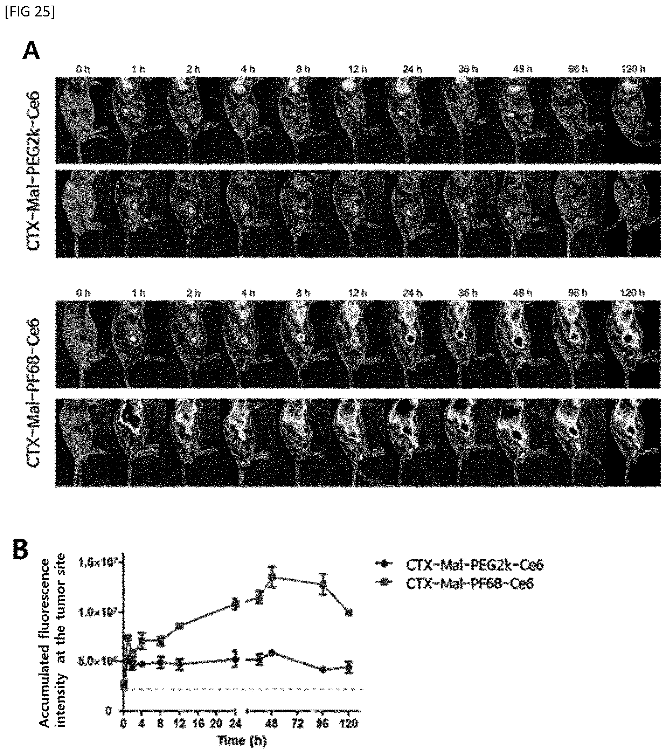

As a result of confirmation, in the cancer tissue of mice injected with cetuximab-maleimide-Pluronic F68-chlorin e6, the fluorescence of a photosensitizer was observed more strongly compared to the cetuximab-maleimide-polyethylene glycol 2k-chlorin e6 (CTX-Mal-PEG2K-Ce6) injection group, which is a comparative group. Even in the results of quantifying the same, it was confirmed that the fluorescence signal was about 2 times that of the comparison group (FIGS. 25A and 25B).

From the above results, it was confirmed that due to the enhanced targeting ability of the antibody-based Pluronic polymer composition, the conjugate was accumulated in cancer tissue for a longer period of time.

### Experimental Example 8: Suppression of in vivo cancer cell growth of antibody-linker-Pluronic-photosensitizer conjugate

Mice (BALB/c nude mice, 5 weeks old) were subcutaneously injected with 1x10⁷ A431 cancer cells, and then when the size of cancer reached 200 mm³ after 15 days, a cetuximab-maleimide-Pluronic F68 or Pluronic F127 conjugate was intravenously injected at a concentration of 0.5 mg/kg based on chlorin e6 five times. Thereafter, the effect of suppressing the growth of cancer cells was confirmed by measuring the size and weight of the cancer tissue once every 2 to 3 days. PBS, cetuximab alone, and cetuximab-maleimide-polyethylene glycol 2K and 6K were used as comparison groups.

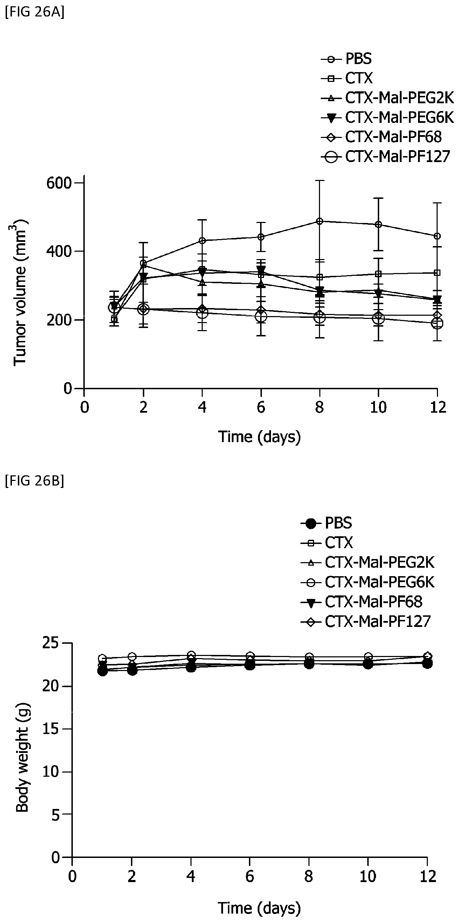

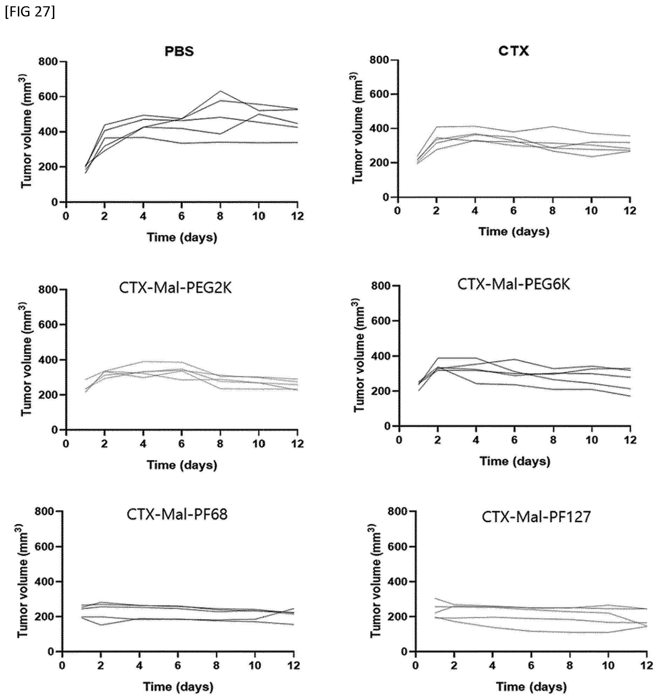

As a result of confirmation, it could be seen that after the first intravenous injection, the tumors grew rapidly in the PBS injection group which is a control. It was confirmed that tumors grew less rapidly in cetuximab (CTX) and cetuximab-maleimide-polyethylene glycol 2K and 6K injection groups (CTX-Mal-PEG2K and CTX-Mal-PEG6K) compared to the control, whereas cancer growth was inhibited in the cetuximab-maleimide-Pluronic F-68 and F127 injection groups (CTX-Mal-PF68 and CTX-Mal-PF127) (FIG. 26A and FIG. 27). Through this, it was confirmed that the cetuximab-maleimide-Pluronic F68 and F127 groups further enhanced the targeting ability of the antibody compared to the polyethylene polymer. In addition, since there was no significant change in the body weight of the mice, it was indirectly confirmed that there was no toxicity in any of the injection groups (FIG. 26B).

### Experimental Example 9: Analysis of ability of antibody-linker-Pluronic-photosensitizer conjugate to produce singlet oxygen

The ability of the cetuximab/trastuzumab-maleimide-Pluronic-photosensitizer conjugate prepared in Example 3 to produce singlet oxygen was analyzed by a fluorescence spectrophotometer (RF) using the antibody-polyethylene glycol-photosensitizer conjugate prepared in Comparative Example 3.

A single oxygen sensor green (SOSG) solution that reacts with singlet oxygen to increase fluorescence was prepared at a concentration of 2 µM, and 1 ml of this solution was mixed with 1 ml of the conjugate sample. A laser with a wavelength of 671 nm was set at an intensity of 50 mW/cm², and the fluorescence of SOSG was measured at Ex 504 nm and Em 525 nm while irradiating the mixed sample with the laser at intervals of 10 seconds.

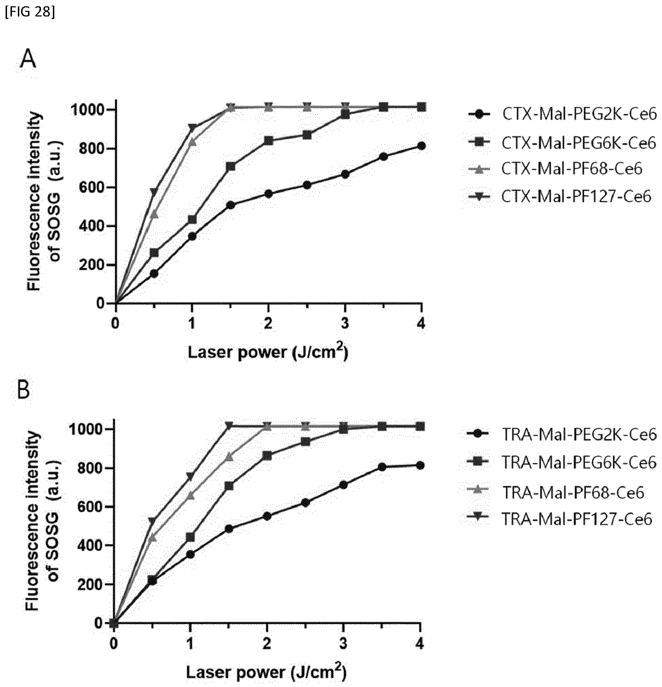

As a result of the measurement, by confirming that the conjugate including Pluronic showed more effective photoactivity than the comparison group, it could be seen that the ability to produce singlet oxygen was excellent (FIG. 28).

### Experimental Example 10: Analysis of ability of antibody-linker-Pluronic-photosensitizer conjugate to produce singlet oxygen

The ability of the cetuximab-maleimide-Pluronic-chlorin e6 conjugate to kill cells was confirmed in cancer cells with different levels of epidermal growth factor receptor (HER2) expression.

After NIH-3T3 cells, which are normal cells and do not have an epidermal growth factor receptor, A2780 cells, which are cancer cells and have a low level of epidermal growth factor receptor expression, and SKOV3 cells, which are cancer cells and have a high level of EGFR expression, were each cultured, each type of cell was treated with a cetuximab-maleimide-Pluronic-chlorin e6 conjugate for 4 hours. Thereafter, the cells were washed with a buffer solution, and were further cultured for 24 hours by adding a new culture solution.

The cultured cells were treated with MTT reagent and cultured for 3 hours, then the culture solution, the MTT reagent and the like were all removed, and dimethyl sulfoxide was added to dissolve formazan formed in the cells. Thereafter, absorbance was measured at 570 nm, and the amount of formed formazan was compared to analyze the viability of each type of cell and the cytotoxicity of the cetuximab-based photosensitizer composition.

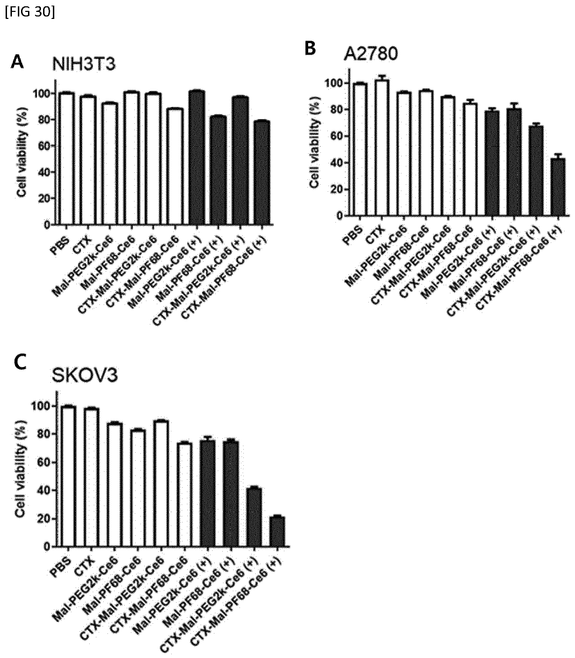

As a result, it was confirmed that even though there was no big difference in cytotoxicity before and after the introduction of cetuximab in NIH-3T3, which is a normal cell (FIG. 30A), cancer showed a big difference in cytotoxicity before and after the introduction of an antibody in proportion to the level of HER2 expression (FIGS. 30A and 30B). In particular, SKOV3 cells, which express the highest epidermal growth factor receptor expression level, showed a self-cytotoxic effect compared to A2780 cells after the introduction of cetuximab, even though they were not irradiated with light.

### Experimental Example 11: Confirmation of cellular phototoxicity of antibody-linker-Pluronic-photosensitizer conjugate

From the previous experimental results, the epidermal growth factor receptor (HER2)-specific distribution pattern of the cetuximab-maleimide-Pluronic-chlorin e6 conjugate prepared in Example 3 was confirmed. Additionally, cells were treated with the conjugate at concentrations that had no cytotoxic effect and irradiated with a laser, and then changes in cell viability were analyzed in normal cells or cancer cells.

After NIH-3T3, which is a normal cell, and epidermal growth factor receptor-expressing cancer cells A2780 and SKOV3 were cultured, the cells were treated with an antibody-based photosensitizer composition and maleimide-polyethylene glycol 2k-chlorin e6 (Mal-PEG 2k-Ce6), which is a comparative group, for 4 hours, irradiated with a laser at an intensity of 2 J/cm², and then cultured again in an incubator for 1 day.

The cultured cells were treated with MTT reagent and cultured for 3 hours, then the culture solution, the MTT reagent and the like were all removed, and dimethyl sulfoxide was added to dissolve formazan formed in the cells. Thereafter, absorbance was measured at 570 nm, and the amount of formed formazan was compared to analyze the viability of each type of cell and the cytotoxicity of the antibody-based photosensitizer composition.

As a result, it was confirmed that in NIH-3T3, which is a normal cell, no phototoxicity appeared before or after the introduction of cetuximab (FIG. 30A), whereas in cancer cells, phototoxicity was increased according to HER2 expression level, cetuximab treatment, and laser irradiation (FIGS. 30B and 30C).

## Claims

1. A conjugate comprising: (a) an antibody for treating cancer;
(b) a linker linked to the antibody via a covalent bond; and
(c) a block copolymer comprising poly(ethylene oxide) (PEO) and poly(propylene oxide) (PPO), which are linked to the linker via a covalent bond.

2. The conjugate of claim 1, wherein the antibody for treating cancer in (a) is selected from the group consisting of an animal-derived antibody, a chimeric antibody, a humanized antibody, and a human antibody.

3. The conjugate of claim 1, wherein the linker in (b) is selected from the group consisting of maleimide, succinic anhydride and N-hydroxysuccinimide ester.

4. The conjugate of claim 1, wherein the block copolymer comprising PEO and PPO in (c) is selected from the group consisting of poloxamer 68, poloxamer 124, poloxamer 127, poloxamer 184, poloxamer 185, poloxamer 188, poloxamer 237, poloxamer 338 and poloxamer 407.

5. The conjugate of claim 4, wherein the block copolymer comprising PEO and PPO is poloxamer 188 or poloxamer 407.

6. The conjugate of claim 1, wherein the covalent bond is selected from the group consisting of an amide bond, a carbonyl bond, an ester bond, a thioester bond, a sulfonamide bond and a urethane bond.

7. The conjugate of claim 1, wherein a low molecular weight compound further binds to one end of the conjugate.

8. The conjugate of claim 7, wherein the low molecular weight compound is an anticancer agent or a photosensitizer.

9. The conjugate of claim 8, wherein the photosensitizer is selected from the group consisting of chlorins, bacteriochlorins, porphyrins, porphycenes and phthalocyanines.

10. The conjugate of claim 9, wherein the chlorin photosensitizer is chlorin e6.

11. A pharmaceutical composition for use in treating cancer, comprising the conjugate of claim 1 as an active ingredient.

12. The pharmaceutical composition of claim 11, wherein the cancer expresses a gene selected from the group consisting of an epidermal growth factor receptor, human epidermal growth factor receptor 2, programmed death-ligand 1 and vascular endothelial growth factor receptor 2 on the surfaces of cancer cells.

13. A method for treating cancer, the method comprising administering the pharmaceutical composition of claim 11 to an individual in need of treatment.
